# EUROPEAN PATENT SPECIFICATION

(11) **EP 1 043 981 B1**
(45) Date of publication and mention of the grant of the patent: **16.04.2003**
(21) Application number: 99900369.2
(22) Date of filing: 04.01.1999
(51) Int. Cl.: A61K 38/46, A61P 3/04, A61K 45/06, A61K 31/78, A61K 31/785

(54) **FAT-BINDING POLYMERS COMBINED WITH LIPASE INHIBITORS FOR THE TREATMENT OF OBESITY**
FETTBINDENDE POLYMERE-LIPASEHEMMER KOMBINATIONEN ZUR BEHANDLUNG VON FETTLEIBIGKEIT
POLYMERES FIXANT LA GRAISSE EN COMBINAISON AVEC DES INHIBITEURS DES LIPASES POUR LE TRAITEMENT DE L'OBESITE

(30) Priority: 09.01.1998 US 4963; 05.10.1998 US 166453
(43) Date of publication of application: 18.10.2000
(73) Proprietor: Geltex Pharmaceuticals, Inc., Waltham, MA 02451 (US)
(72) Inventor: MANDEVILLE, W., Harry, III., Lynnfield, MA 01940 (US); WHITESIDES, George, M., Newton, MA 02458 (US); HOLMES-FARLEY, Stephen, Randall, Arlington, MA 02476 (US)
(74) Representative: Stevens, Ian Edward
(86) International application number: US9900028
(87) International publication number: WO99034787

(56) References cited:
- EP-A- 0 050 347
- WO-A-89/07455
- FR-A- 2 081 400
- US-A- 4 211 765
- US-A- 4 432 968
- PATENT ABSTRACTS OF JAPAN vol. 095, no. 002, 31 March 1995 (1995-03-31) & JP 06 321787 A (SEKISUI CHEM CO LTD), 22 November 1994 (1994-11-22)
- ATKINSON ET AL: "Combined Drug Treatment of Obesity" OBESITY RESEARCH, vol. 3, no. S4, November 1995 (1995-11), pages 497S-500S, XP002104491

## Description

### BACKGROUND OF THE INVENTION

Human obesity is a recognized health problem with approximately 97 million people considered clinically overweight in the United States. The accumulation or maintenance of body fat bears a direct relationship to caloric intake. Therefore, one of the most common methods for weight control to combat obesity is the use of relatively low-fat, low calorie diets, that is, diets containing less fat and calories than a "normal diet" or that amount generally consumed by the patient.

The presence of fats in a great many food sources greatly limits the food sources which can be used in a low-fat diet. Additionally, fats contribute to the flavor, appearance and physical characteristics of many foodstuffs. As such, the acceptability of low-fat diets and the maintenance of such diets are difficult.

Various chemical approaches have been proposed for controlling obesity. Anorectic agents, such as dextroamphetamine, the combination of the non-amphetamine drugs phentermine and fenfluramine ("Phen-Fen") and dexfenfluramine (Redux) alone, are associated with serious side effects. Indigestible materials such as OLESTRA™, mineral oil or neopentyl esters (see U.S. Patent No. 2,962,419) have been proposed as substitutes for dietary fat. Garcinia acid and derivatives thereof have been described as treating obesity by interfering with fatty acid synthesis. Swellable crosslinked vinyl pyridine resins have been described as appetite suppressants via the mechanism of providing non-nutritive bulk, as in U.S. Patent 2,923,662. Surgical techniques, such as temporary ileal bypass surgery, are employed in extreme cases.

However, methods for treating obesity, such as those described above, have serious shortcomings with controlled diet remaining the most prevalent technique for controlling obesity. As such, new methods for treating obesity are needed.

US 4432968 discloses pharmacologically-acceptable fat imbibing polymers which can be introduced into the gastrointestinal tract of animals in order to control body weight.

Patent Abstracts of Japan, volume 95, no 2, 31 March 1995 and JP-A-06-321787 describe agents for inhibiting the absorption of carbohydrates which comprise a polyamine.

FR-A-2081400 teaches pharmaceutical preparations containing polyamines for absorbing biliary acids and triglycerides, inhibiting lipases, and reducing amounts of cholesterol and/or triglycerides.

US 4211765 discloses a method of weight control in which polymers of unsaturated carboxylic acid derivatives, particularly cationic polymers such as dialkylaminoalkyl imides of alkene/ maleic anhydride copolymers, are orally administered to interfere with assimilation of fat.

### SUMMARY OF THE INVENTION

In a first aspect, the invention provides a pharmaceutical composition or kit of parts for treating obesity in a mammal by oral administration to the mammal comprising an effective amount of one or more fat-binding polymers and one or more lipase inhibitors.

In another aspect, the invention provides the use of an effective amount of one or more fat-binding polymers in the manufacture of a medicament for simultaneous or sequential administration with one or more lipase inhibitors for treating obesity in a mammal by oral administration.

In yet another aspect, the invention provides a pharmaceutical composition or kit of parts for reducing the absorption of dietary fat in a mammal by oral administration to the mammal comprising a therapeutic amount of one or more fat-binding polymers in combination with one or more lipase inhibitors.

In a further aspect, the invention provides the use of a therapeutic amount of one or more fat-binding polymers in the manufacture of a medicament for treating steatorrhea by oral administration.

In a yet further aspect, the invention provides the use of an effective amount of one or more fat-binding polymers in the manufacture of a medicament for simultaneous or sequential administration with one or more lipase inhibitors for treating hypertriglyceridemia in a mammal by oral administration.

The administration of a fat-binding polymer of the invention facilitates the excretion of fat from the body without digestion, with minimal side effects and low toxicity. The fat-binding polymers are administered in combination with a therapeutically effective amount of a lipase inhibitor, such as the pancreatic lipase inhibitors described in U.S. Patent No. 4,598,089 to Hadvary *et al.* The combination administration can reduce undesirable side effects often encountered when lipase inhibitors, in particular, the pancreatic lipase inhibitors lipstatin and tetrahydrolipstatin are administered alone. For example, a serious side effect resulting from the administration of a lipase inhibitor is steatorrhea, or fatty stools.

The fat-binding polymers of the invention comprise at least one fat-binding region. A fat-binding region can include a region having a positive charge, a region which is hydrophobic or a region having a positive charge and which is hydrophobic.

In one embodiment, the fat-binding polymer is an aliphatic polymer selected from the group consisting of polyalkylacrylates, polyacrylamides, polyalkylmethacrylates, polymethacrylamides, poly-N-alkylacrylamides, poly-N-alkylmethacrylamides, substituted derivatives thereof and copolymers thereof. For example, the substituted derivatives of the polymers can be characterized by one or more substituents, such as substituted or unsubstituted, saturated or unsaturated alkyl, and substituted or unsubstituted aryl groups. Suitable substituents to employ on the alkyl or aryl groups include, but are not limited to, cationic or neutral groups, such as alkoxy, aryl, aryloxy, aralkyl, halogen, amine, and ammonium groups. For example, the polymer can be poly(dimethylamino propylacrylamide), poly(trimethylammonium ethylacrylate), poly(trimethylammonium ethyl methacrylate), poly(trimethylammonium propyl acrylamide), poly(dodecyl acrylate), poly(octadecyl acrylate), poly(octadecyl methacrylate) and copolymers thereof.

In another embodiment, the fat binding polymer is a synthetic amine polymer. Amine polymers suitable for use in the invention include, but are not limited to, poly(allylamine), polyethyleneimine, poly(vinylamine), poly(diallylamine), and poly(diallylmethylamine).

In yet another embodiment, the fat-binding polymer is a hydroxyl-containing polymer, for example, poly(vinylalcohol).

In a specific embodiment, the fat-binding polymer is an amine-containing polymer wherein one or more hydrophobic regions are bound to a portion of the amine nitrogens of the amine polymer. In a particular embodiment, between 1 and 60 percent of the amine nitrogens are substituted, preferably between 1 and 30 percent.

In another embodiment, the hydrophobic region of the fat-binding polymer can include a hydrophobic moiety, for example, a substituted or unsubstituted, normal, branched or cyclic alkyl group having at least four carbons. In a particular embodiment, the hydrophobic moiety is an alkyl group of between four and thirty carbons.

In another embodiment, the hydrophobic region is a quaternary amine-containing moiety having a terminal hydrophobic substituent. Suitable hydrophobic regions which can include a hydrophobic moiety and/or a quaternary amine-containing are described herein and in U.S. Patent Nos. 5,607,669, 5,679,717 and 5,618,530.

In yet another embodiment, the fat-binding polymer is substituted by a lipase inhibitor such as those described in U.S.S.N. 09/005,379 filed on January 9, 1998, and U.S.S. N. 09/166,510 filed on October 5, 1998.

The polymers of the present invention offer desirable pharmacological properties such as excellent fat binding properties and low toxicity. In addition, when the fat-binding polymers are administered in combination with lipase inhibitors, as described herein, undesirable side effects experienced, such as steatorrhea, when the lipase inhibitors are administered alone can be lessened.

### DETAILED DESCRIPTION OF THE INVENTION

"Lipases" as that term is used herein, are ubiquitous enzymes which hydrolyze ester bonds in neutral lipids. Examples of lipases include, but are not limited to, pancreatic and gastric lipases. The preferred substrates of lipases are insoluble in water. Lipases exhibit maximal activity in the presence of lipid/water interfaces. For example, pancreatic lipase, which is the key enzyme of dietary triglyceride absorption, exerts it activity at the water/lipid interface, in conjunction with bile salts and co-lipase.

"Lipase inhibitor" as that term is used herein refers to compounds which are capable of inhibiting the action of lipases, for example, gastric and pancreatic lipases. Lipstatin and its tetrahydro derivative, Tetrahydrolipstatin, as described in U.S. Patent No. 4,598,089 to Hadvary *et al.*, are potent inhibitors of both gastric and pancreatic lipases, as well as cholesterol ester hydrolase. Lipstatin is a natural product of microbial origin, and tetrahydrolipstatin is the result of catalytic hydrogenation of lipstatin. Other lipase inhibitors include a class of compound commonly referred to as Panclicins. Panclicins are analogues of Tetrahydrolipstatin (See e.g., Mutoh, M., *et al.*, "Panclicins, Novel Pancreatic Lipase Inhibitors, II. Structural Elucidation," *The Journal of Antibiotics, 47*(12): 1376-1384 (1994)).

"Fat-binding polymers", as that term is used herein, are polymers which absorb, bind or otherwise associate with fat thereby inhibiting (partially or completely) fat digestion, hydrolysis, or absorption in the gastrointestinal tract and/or facilitating the removal of fat from the body prior to digestion. The fat-binding polymers comprise one or more fat-binding regions. "Fat-binding regions", as defined herein can include a positively charged region, a hydrophobic region, or a region which is both positively charged and hydrophobic.

"Fats", as that term is used herein, are solids or liquid oils generally consisting of glycerol esters of fatty acids. Sources of fats include both animal and vegetable fats, for example, triglyceride esters of saturated and/or unsaturated fatty acids, free fatty acids, diglycerides, monoglycerides, phospholipids and cholesterol esters are fats, as defined herein.

A variety of polymers can be employed in the invention described herein. The polymers are synthetic polymers which can be aliphatic, or aromatic. However, aliphatic and synthetic polymers are preferred. A "synthetic polymer", as that term is employed herein, is a polymer which is not obtainable from a natural source either directly or through a minor derivatization of the naturally occurring form. Further, the polymer can be hydrophobic, hydrophilic or copolymers of hydrophobic and/or hydrophilic monomers. The polymers can be manufactured from olefinic or ethylenic monomers (such as vinylalcohol, allylamine or acrylic acid) or condensation polymers.

For example, the polymers can include polyvinylalcohol, polyvinylamine, poly-N-alkylvinylamine, polyallylamine, poly-N-alkylallylamine, polydiallylamine, poly-N-alkyldiallylamine, polyalkylenimine, other polyamines, polyethers, polyamides, polyacrylic acids, polyalkylacrylates, polyacrylamides, polymethacrylic acids, polyalkylmethacrylates, polymethacrylamides, poly-N-alkylacrylamides, poly-N-alkylmethacrylamides, polystyrene, polyvinylnaphthalene, polyethylvinylbenzene, polyaminostyrene, polyvinylbiphenyl, polyvinylanisole, polyvinylimidazolyl, polyvinylpyridinyl, polydimethylaminomethylstyrene, polytrimethylammonium ethyl methacrylate, polytrimethylammonium ethyl acrylate, and substituted derivatives of the above (e.g., fluorinated monomers thereof) and copolymers thereof. In addition, the polymers can be further characterized by one or more substituents such as substituents, such as substituted and unsubstituted, saturated or unsaturated alkyl, and substituted or unsubstituted aryl groups. Suitable groups to employ include cationic or neutral groups, such as alkoxy, aryl, aryloxy, aralkyl, halogen, amine, and ammonium groups.

Particularly preferred polymers include polyalkylacrylates, polyacrylamides, polyalkylmethacrylates, polymethacrylamides, poly-N-alkylacrylamides, poly-N-alkylmethacrylamides and copolymers thereof. These polymers can be further characterized by one or more substituents, such as substituted or unsubstituted, saturated or unsaturated alkyl, and substituted or unsubstituted aryl groups. Suitable substituents include cationic or neutral groups, such as alkoxy, aryl, aryloxy, aralkyl, halogen, amine, and ammonium groups, for example.

Other particularly preferred polymers include aliphatic amine polymers, such as polyallylamine, polydiallylamine, polydiallylmethylamine, polyvinylamine, polyethylenimine. In a specific embodiment, the amine polymer comprises one or more hydrophobic regions which are bound to a portion of the amine nitrogens of the amine polymer. In a particular embodiment, between 1 and 60 percent of the amine nitrogens are substituted, preferably between 1 and 30 percent.

In one embodiment, the hydrophobic region of the fat-binding polymer can include a hydrophobic moiety, for example, a substituted or unsubstituted, normal, branched or cyclic alkyl group having at least four carbons. In a specific embodiment, the hydrophobic moiety is an alkyl group of between four and thirty carbons.

In another embodiment, the hydrophobic region is a quaternary amine-containing moiety having a terminal hydrophobic substituent.

In yet another embodiment, the fat-binding region comprises a nitrogen, for example, the nitrogen of an amine, capable of possessing a positive charge under conditions present in the gastro-intestinal tract. For example, a quaternary amine-containing moiety, or the nitrogen of a polyamine.

In yet another embodiment, the fat-binding polymer is a hydroxyl-containing polymer, for example, poly(vinylalcohol) which can comprise further fat-binding regions. For example, the polymer comprises a repeat unit having the formula 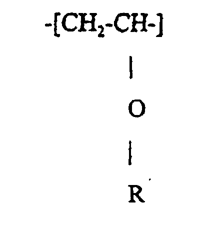 wherein R is a hydrophobic region.

Other polymers and methods of preparation, which can be used in the claimed invention have been reported in the patent literature in, for example, United States Patent Nos. 5,487,888, 5496,545, 5,607,669, 5,618,530, 5,624,963, 5,667,775, and 5,679,717 and co-pending U.S. Applications having Serial Nos 08/471,747, 08/482,969, 081567,933, 08/659,264, 08/823,699, 08/835,857, 08/470,940, 08/461,298, 08/826,197, 08/777,408, 08/927,247, 08/964,956, 08/964,498, and 08/964,536.

The polymer can be linear or crosslinked. Crosslinking can be performed by reacting the copolymer with one or more crosslinking agents having two or more functional groups, such as electrophilic groups, which react with, for example, amine groups to form a covalent bond. Crosslinking in this case can occur, for example, via nucleophilic attack of the polymer amino groups on the electrophilic groups. This results in the formation of a bridging unit which links two or more amino nitrogen atoms from different polymer strands. Suitable crosslinking agents of this type include compounds having two or more groups selected from among acyl chloride, epoxide, and alkyl-X, wherein X is a suitable leaving group, such as a halo, tosyl or mesyl group. Examples of such compounds include, but are not limited to, epichlorohydrin, succinyl dichloride, acryloyl chloride, butanedioldiglycidyl ether, ethanedioldiglycidyl ether, pyromellitic dianhydride, and dihaloalkanes. These crosslinking agents are referred to herein as multifunctional crosslinking agents.

The polymer composition can also be crosslinked by including a multifunctional co-monomer as the crosslinking agent in the polymerization reaction mixture. A multifunctional co-monomer can be incorporated into two or more growing polymer chains, thereby Crosslinking the chains. Suitable multifunctional co-monomers include, but are not limited to, diacrylates, triacrylates, and tetraacrylates, dimethacrylates, diacrylamides, and dimethacrylamides. Specific examples include ethylene glycol diacrylate, propylene glycol diacrylate, butylene glycol diacrylate, ethylene glycol dimethacrylate, butylene glycol dimethacrylate, methylene bis(methacrylamide), ethylene bis(acrylamide), ethylene bis(methacrylamide), ethylidene bis(acrylamide), ethylidene bis(methacrylamide), pentaerythritol tetraacrylate, trimethylolpropane triacrylate, bisphenol A dimethacrylate, and bisphenol A diacrylate. Other suitable multifunctional monomers include polyvinylarenes, such as divinylbenzene.

The amount of cross-linking agent is typically between 0.5 and 25 weight % based on the combined weight of crosslinking agent and monomers, with 1-20% being preferred. Typically, the amount of cross-linking agent that is reacted with the polymer, when the crosslinking agent is a multifunctional agent, is sufficient to cause between 0.1 and 20 percent of the nucleophiles present on the monomer, for example, an amine to react with the crosslinking agent. In a preferred embodiment, between 3 and 15 percent of the nucleophilic sites, for example, amines react with the multifunctional crosslinking agent.

The hydrophobic region or regions of the fat-binding polymers include but are not limited to, for example, a hydrophobic moiety such as a substituted or unsubstituted, normal, branched or cyclic alkyl group having at least four carbons. For example, a hydrophobic moiety such as an alkyl group of at least four carbons can be bound to the fat-binding polymer, for example, through an amine of the fat-binding polymer.

A "hydrophobic moiety", as the term is used herein, is a moiety which, as a separate entity, is more soluble in octanol than water. For example, the octyl group (C₈H₁₇) is hydrophobic because its parent alkane, octane, has greater solubility in octanol than in water. The hydrophobic moieties can be a saturated or unsaturated, substituted or unsubstituted hydrocarbon group. Such groups include substituted and unsubstituted, normal, branched or cyclic alkyl groups having at least four carbon atoms, substituted or unsubstituted arylalkyl or heteroarylalkyl groups and substituted or unsubstituted aryl or heteroaryl groups. Preferably, the hydrophobic moiety includes an alkyl group of between four and thirty carbons. Specific examples of suitable hydrophobic moieties include the following alkyl groups n-hexyl, n-heptyl, n-octyl, n-nonyl, n-decyl, n-undecyl, n-dodecyl, n-tetradecyl, n-octadecyl, 2-ethylhexyl, 3-propyl-6-methyl decyl and combinations thereof. Other examples of suitable hydrophobic moieties include haloalkyl groups of at least six carbons (e.g., 10-halodecyl), hydroxyalkyl groups of at least six carbons (e.g., 11-hydroxyundecyl), and aralkyl groups (e.g., benzyl).

The positively charged region or regions of the fat-binding polymers include but are not limited to, for example, an amine nitrogen capable of possessing a positive charge under conditions present in the gastro-intestinal tract and a quaternary amine-containing moiety. Suitable quaternary amine-containing moieties include alkyl trialkylammonium groups also referred to as ammonioalkyl groups. The term, "ammonioalkyl", as used herein, refers to an alkyl group which is substituted by a nitrogen bearing three additional substituents. Thus, the nitrogen atom is an ammonium nitrogen atom which bears an alkylene substituent, which links the ammonium nitrogen atom to the polymer, and three additional terminal alkyl substituents having from one to twenty-four carbons. A "terminal substituent" of the quaternary amine-containing moiety, as the term is employed herein, is any one of the three substituents on the quaternary amine nitrogen which is not the carbon chain between the polymer backbone and the nitrogen of the quaternary ammonium center. In a specific embodiment, the polymer is an amine polymer and the alkylene group links the ammonium nitrogen atom to the nitrogen atom of the polymer. It is to be understood that multiple moieties can be bound to the same amine and/or different amines of the polymer composition.

In another embodiment, the quaternary amine-containing moiety can bear at least one terminal hydrophobic alkyl substituent, such as an alkyl group having between four and twenty-four carbons, thereby providing both a hydrophobic region and a positively charged region in combination.

An ammonioalkyl group will further include a negatively charged counterion, such as a conjugate base of a pharmaceutically acceptable acid. Examples of suitable counterions include Cl⁻, PO₄⁻, Br⁻, CH₃SO₃⁻, HSO₄⁻, SO₄²⁻, HCO₃⁻, CO₃²⁻, acetate, lactate, succinate, propionate, butyrate, ascorbate, citrate, maleate, folate, an amino acid derivative, and a nucleotide.

Suitable ammonioalkyl groups are of the general formula: 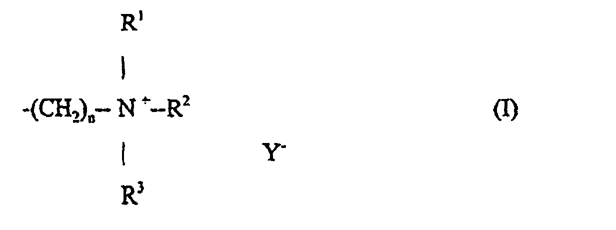 wherein, R¹, R² and R³ represent an alkyl group, wherein each R¹-R³, independently, is a normal or branched, substituted or unsubstituted alkyl group having a carbon atom chain length of between one to twenty-four carbon atoms, n is an integer having a value of two or more and Y is a negatively charged counterion. In a particular embodiment, R¹, R² and R³ are all methyl groups and n is an integer between 2 and 12 (e.g., 2,3,4,5,6,7,8,9,10,11, or 12).

The alkyl group, which provides the alkylene linking group between the polymer, for example, and the amine of the amine-containing monomer or repeat unit, and the ammonium nitrogen of the alkyl trialkylammonium group, is two or more carbon atoms in length. Examples of preferred alkylene linking groups are ethyl, propyl, butyl, pentyl, hexyl, octyl, and decyl groups. Example of suitable quaternary amine-containing moieties include, but are not limited to:
3-(trimethylammonio)propyl; 4-(trimethylammonio)butyl; 6-(trimethylammonio)hexyl; 8-(trimethylammonio)octyl; 10-(trimethylammonio)decyl; 12-(trimethylammonio)dodecyl and combinations thereof. A particularly preferred amine-containing moiety is a 6-(trimethylammonio)hexyl group.

Alternatively, a quaternary amine-containing moiety and a hydrophobic moiety are present in the same substituent, thereby providing both a positively charged and hydrophobic region in combination. For example, the quatemary amine nitrogen or ammonium nitrogen of the quaternary amine-containing moiety is bound to the polymer backbone by an alkylene having two or more carbons. However, at least one of the three terminal substituents (R¹, R² and R³) of the ammonium nitrogen is a hydrophobic alkyl group having from four to twenty-four carbors. The remaining terminal substituents are each independently a normal or branched, substituted or unsubstituted alkyl group having from one to twenty-four carbons or a hydrogen atom. In another embodiment, at least two of the three terminal substituents can be hydrophobic alkyl groups having from four to twenty-four carbons, the remainder having from one to twenty-four carbons or a hydrogen atom. In a further embodiment, all three of the terminal substituents can be hydrophobic alkyl groups having from six to twenty-four carbons.

A "hydrophobic alkyl group", as that term is employed herein, includes a substituted or unsubstituted alkyl group having from four to twenty-four carbons and which is hydrophobic, as earlier defined. The hydrophobic alkyl group can be, for example, a normal or branched, substituted or unsubstituted alkyl group having from six to twenty-four carbons.

Particular examples of quaternary amine-containing moieties, which provide both a hydrophobic and quaternary amine-containing substituent, include, but are not limited to:
4-(dioctylmethylammonio)butyl; 3-(dodecyldimethylammonio)propyl;
3-(octyldimethylammonio)propyl; 3-(decyldimethylammonio)propyl;
5-(dodecyldimethylammonio)pentyl; 6-(dimethyldecylammonio)hexyl;
6-(decyldimethylammonio)hexyl; 3-(tridecylammonio)propyl;
3-(docosyldimethylammonio)propyl; 6-(docosyldimethylammonio)hexyl;
4-(dodecyldimethylammonio)butyl; 3-(octadecyldimethylammonio)propyl;
3-(hexyldimethylammonio)propyl; 3-(methyldioctylammonio)propyl;
3-(didecylmethylammonio)propyl; 3-(heptyldimethylammonio)propyl;
3-(dimethylnonylammonio)propyl; 6-(dimethylundecylammonio)hexyl;
4-(heptyldimethylammonio)butyl; 4-(dioctylmethylammonio)butyl;
6-(octyldimethylammonio)hexyl; 12-(decyldimethylammonio)dodecyl;
3-(dimethylundecylammnio)propyl; and 3-(tetradecyldimethylammonio)propyl.

Other suitable quaternary amine-containing moieties include secondary and tertiary analogs, such as 4-(dioctylmethylammonio)4-methylbutyl and 4-(dioctylmethylammonio)-4,4-dimethylbutyl.

The fat-binding polymers of the invention can be formed, for example, by reacting a polymer, which can be linear or crosslinked, with a suitable alkylating agent or by polymerizing an alkylated monomer.

An "acylating agent", as that term is employed herein, means a reactant that, when reacted with a monomer or a copolymer characterized by a repeat unit of the invention and having a nucleophilic site capable of reaction with the acylating agent, causes an acyl substituent, in particular a hydrophobic acyl substituent, as described herein, to be covalently bound to one or more of sites on the fat-binding polymer, for example, the amine nitrogen atoms or hydroxyl oxygens of an amine-containing or hydroxyl-containing monomer or polymer, respectively. Further, when multiple substituents are employed, they can be bound to the same and/or different nucleophilic sites of the fat-binding polymer, for example, the same and/or different amine nitrogens of an amine-containing fat-binding polymer or hydroxyl oxygen of a hydroxyl-containing polymer.

Suitable acylating agents are compounds comprising an acyl group or acyl derivative, for example an anhydride . For example, when the acylating agent is acetic anhydride the nucleophile is modified by addition of an acetyl group. Acylating agents suitable for the addition of a hydrophobic moiety contain an acyl group having at least four carbon atoms, which is bonded to a leaving group such as a halo (e.g., chloro, bromo or iodo). Activated esters are also suitable acylating agents. Examples of suitable acylating agent which provide a hydrophobic moiety include acyl halides having at least four carbon atoms, such as butyryl halide, valeryl halide, hexanoyl halide, heptanoyl halide, octanoyl halide, nonanoyl halide, decanoyl halide, undecanoyl halide, and combinations thereof.

An "alkylating agent", as that term is employed herein, means a reactant that, when reacted with a monomer or a copolymer characterized by a repeat unit of the invention and having a nucleophilic site capable of reaction with the alkylating agent, causes a hydrophobic substituent, as described herein, to be covalently bound to one or more of sites on the fat-binding polymer, for example, the amine nitrogen atoms or hydroxyl oxygens of an amine-containing or hydroxyl-containing monomer or polymer, respectively. Further, when multiple substituents are employed, they can be bound to the same and/or different nucleophilic sites of the fat-binding polymer, for example, the same and/or different amine nitrogens of an amine-containing fat-binding polymer or hydroxyl oxygen of a hydroxyl-containing polymer.

Suitable alkylating agents are compounds comprising an alkyl group or alkyl derivative, having at least four carbon atoms, which is bonded to a leaving group such as a halo (e.g., chloro, bromo or iodo), tosylate, mesylate or epoxy group).

Examples of suitable alkylating agents which provide a hydrophobic moiety include alkyl halides having at least four carbon atoms, such as n-hexyl halide, n-heptyl halide, n-octyl halide, n-nonyl halide, n-decyl halide, n-undecyl halide, n-dodecyl halide, n-tetradecyl halide, n-octadecyl halide, and combinations thereof. Other examples include: a dihaloalkane that includes an alkyl group of at least four carbons (e.g., a 1,10-dihalodecane); a hydroxyalkyl halide having at least four carbon atoms (e.g., an 11-halo-l-undecanol); an aralkyl halide (e.g., a benzyl halide); an alkyl epoxy ammonium salt having at least six carbons (e.g., glycidylpropyl-trimethylammonium salts) and epoxyalkylamides having at least six carbons (e.g., N-(2,3-epoxypropyl) butyramide or N-(2,3-epoxypropyl) hexanamide). Preferred halogen components of the alkyl halides are bromine and chlorine. Particularly preferred alkylating agents which, when reacted with the polymer composition, will cause formation of an amine polymer reaction product that includes a first substituent, are 1-bromodecane and 1-chlorooctane.

Examples of suitable alkylating agents which can provide a quaternary amine-containing moiety have the general formula: 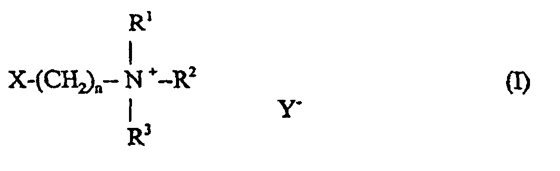 wherein,
- R¹, R², and R³: represent an alkyl group, wherein each R independently is a normal or branched, substituted or unsubstituted alkyl group having a carbon atom chain length of between one to twenty four carbon atoms,
n is an integer having a value of two or more,
X is a leaving group as earlier described, and
Y is a negatively charged counterion.

When at least one of the three terminal substituents of the quaternary amine alkylating agent is a hydrophobic alkyl group having from four to twenty-four carbons, the alkylating agent therefore provides both a hydrophobic moiety and a quaternary amine-containing moiety. The alkylene group in this instance is three or more carbon atoms in length.

Particular examples of quaternary ammonium compounds suitable as alkylating agents include the following:
(4-bromobutyl)dioctylmethylammonium bromide;
(3-bromopropyl)dodecyldimethylammonium bromide;
(3-chloropropyl)dodecyldimethylammonium bromide;
(3-chloropropyl)decyldimethylammonium bromide;
(5-tosylpentyl)dodecyldimethylammonium bromide;
(6-bromohexyl)dimethyldecylammonium bromide;
(12-bromododecyl)decyldimethylammonium bromide;
(3-bromopropyl)tridecylammonium bromide;
(3-bromopropyl)docosyldimethylammonium bromide;
(6-bromohexyl)docosyldimethylammonium bromide;
(4-chlorobutyl)dodecyldimethylammonium bromide;
(3-chloropropyl)octadecyldimethylammonium bromide;
(3-bromopropyl)octyldimethylammonium bromide;
(4-iodobutyl)dioctylmethylammonium bromide;
(2,3-epoxy propyl)decyldimethylammonium bromide; and
(6-bromohexyl)docosyldimethyammonium bromide.

Other suitable alkylating agents include secondary and tertiary analogs, such as (3-bromobutyl)dioctylmethylammonium bromide and (3-chloro-3,3-dimethyl propyl)dioctylmethylammonium bromide.

Examples of suitable alkyl trimethylammonium alkylating agents include alkyl halide trimethylammonium salts, such as:
(4-halobutyl)trimethylammonium salt; (5-halopentyl)trimethylammonium salt;
(6-halohexyl)trimethylammonium salt; (7-haloheptyl)trimethylammonium salt;
(8-halooctyl)trimethylammonium salt; (9-halononyl)trimethylammonium salt;
(10-halodecyl) trimethylammonium salt; (11-haloundecyl)trimethylammonium salt;
(12-halododecyl)trimethylammonium salt; and combinations thereof. A particularly preferred quaternary amine-containing alkylating agent is (6-bromohexyl)-trimethylammonium bromide.

In another embodiment, the fat-binding polymer can have a lipase inhibitor covalently bound to the polymer as described in U.S.S.N. 09/005,379 filed on January 9, 1998, and U.S.S.N. 09/166,510 filed on October 5, 1998. In a further embodiment, the fat-binding polymer can be administered in combination with a lipase inhibitor which is convalently bound to a polymer as described in U.S.S.N. 09/005,379 filed on January 9, 1998, and U.S.S.N. 09/166,510 filed on October 5, 1998.

As used herein, the terms "therapeutically effective amount" and "therapeutic amount" are synonymous. The terms refer to an amount which is sufficient to treat obesity, reduce the absorption of fat, facilitate the removal of fat prior to digestion or treat hypertriglyceridemia. The dosage of fat-binding polymer administered to the patient will vary depending among other things on the weight of the patient and the general health of the patient. The dosage can be determined with regard to established medical practice. The amount of fat-binding polymer administered can be in the range of from
0.01 mg/kg of body weight/day to 1g/kg of body weight/day. The amount of lipase inhibitor which can be administered in combination with the fat-binding polymers of the invention can be determined with regard to accepted medical practice.

As disclosed above, the fat-binding polymer is generally administered in combination with a lipase inhibitor, as described herein. The term "in combination" in this context includes both simultaneous or sequential administration (either type of compound first) of the fat-binding polymer and lipase inhibitor. The fat-binding polymer and lipase inhibitor, when used in combination, can be employed together in the same dosage form or in separate dosage forms taken at the same time or within a time period, wherein both the fat-binding polymer and lipase inhibitor are present in a therapeutically effective amount.

The fat-binding polymers of the invention can be formulated using conventional inert pharmaceutical adjuvant materials into dosage forms which are suitable for oral administration. The oral dosage forms include tablets, capsules, suspension, solutions, and the like. The identity of the inert adjuvant materials which are used in formulating the fat-binding polymers of the invention will be immediately apparent to persons skilled in the art. These adjuvant materials, either inorganic or organic in nature, include, for example, gelatin, albumin, lactose, starch, magnesium stearate, preservatives (stabilizers), melting agents, emulsifying agents, salts, and buffers.

In patients with hypertriglyceridemia it is to be understood that the patient does not necessarily suffer from hypercholesterolemia.

### EXEMPLIFICATIONS

### EXAMPLE 1: SYNTHESIS OF DIALLYLAMINE-HCl (DAA-HCl) SOLUTION

Diallylamine (DAA) (2000.3 g) was added slowly over a period of 2 days to concentrated HCl (2035.6 g). The temperature of the reaction was maintained below 10°C by cooling the flask in an ice-salt-water bath, and by adjusting the addition rate. The room temperature pH of the resulting DAA-HCl solution (68.16% DAA-HCl) was 0.005.

### EXAMPLE 2: POLYMERIZATION OF DIALLYLAMINE-HCl

To a 12-liter, 4-necked, round-bottomed flask equipped with an overhead stirrer and an air condenser was added DAA-HCl (3667.8 g of a 68.16% solution, and deionized water (4665.5 g). The resulting solution had a pH of 0.741. To the flask was added NaOH (66.8 g of a 50% aqueous solution). The resulting solution had a pH of 2.554. Nitrogen gas was bubbled through the solution, via a stainless steel needle, with stirring, and venting on top of the air condenser for 2 hours. The nitrogen line was put on top of the air condenser with positive pressure from a mineral oil bubbler. To the flask was added 125.0 g of freshly made 20% V-50 (Wako Chemicals USA, Inc., Richmond, VA) in deionized water. This was added via syringe through a septum. The V-50 solution was not degassed with nitrogen. The solution was heated to 60°C over a period of 1 hour and 8 minutes, with a heating mantle connected to a J-Kem temperature controller. The solution was heated at 60°C for 18 hours. After the first 18 hour heating period, the reaction solution was allowed to cool down slowly to 49°C, and to the flask was added 125.0 g of freshly made 20% V-50 in deionized water. The solution was heated to 60°C over a period of about 15 minutes, with a heating mantle connected to a J-Kem temperature controller. The solution was heated at 60°C for 18 hours. After the second 18 hour heating period, the reaction solution was allowed to cool down slowly to 40°C, and to the flask was added 125.0 g of freshly made 20% V-50 in deionized water. The solution was heated to 60°C over a period of about 15 minutes, with a heating mantle connected to a J-Kem temperature controller. The solution was heated at 60°C for 18 hours. After cooling to room temperature, the solution was a dark orange viscous, flowable, clear solution. The flask contents were combined with deionized water (4166.7 g). The resulting solution had a pH of 4.4. SEC analysis: Mw 61494 Daltons; Polydispersity 2.43.

### EXAMPLE 3: CROSSLINKING OF A SOLUBLE POLYMER TO OBTAIN A INSOLUBLE GEL; PREPARATION OF 3 MOL% CROSSLINKED POLY(ALLYLAMINE)HCL

Poly(allylamine) Hydrochloride (200 g of 50% aqueous solution, 1.07 mol monomer equivalents) was dissolved in a mixture of ethanol (213 mL) and water (125 mL) in a 1-liter, round-bottomed flask equipped with an overhead mechanical stirrer. The pH of the solution was brought to 10.0 - 10.2 by the addition of NaOH (50% solution). Epichlorohydrin (2.97 g, 32.07 mmol) was then added to the rapidly stirred solution in one portion. This mixture was stirred at room temperature (19-22°C) until a gel formed (approx. 30 min), then stirring was suspended and the mixture was allowed to sit at room temperature for 20 hours. After the 20 hour reaction time had elapsed, the gel was transferred into a 5-liter bucket with 3 liters of deionized water. The mixture was then stirred with an overhead mechanical stirrer until the gel was well dispersed in solution. The pH was then adjusted to < 1 using concentrated HCI. The mixture was then vacuum filtered through Whatman 541 filter paper. The filtered polymer gel was then collected and purified by suspension into 4 liters of deionized water followed by vacuum filtration through Whatman 541 filter paper. The procedure of suspension into deionized water followed by vacuum filtration was repeated several times until the conductivity of the suspended polymer gel was < 0.5 mS/cm. After the final vacuum filtration, the polymer gel was transferred into several Pyrex drying trays and placed into a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28mm Hg for at least 16 hours. Yield = 83%.

**Table 1.**

| Epichlorohydrin Crosslinking Reactions Using a Procedure Similar to Example 3 | | |
|---|---|---|
| Example | Polyamine | mol% Xlink |
| 4 | Poly(allylamine) HCl | 6 |
| 5 | Poly(allylamine) HCl | 9 |
| 6 | Poly(allylamine) HCl 10 mol% C 12H 25 (prepared according to Example 82) | 3 |
| 7 | Polyethylenimine | 3 |
| 8 | Polyethylenimine | 6 |
| 9 | Poly(diallylamine) HCI | 3 |
| 10 | Poly(diallylamine) HCl | 4.5 |
| 11 | Poly(diallylamine) HCl | 6 |
| 12 | Poly(diallylmethylamine) HCl | 4.5 |
| 13 | Poly(vinylamine) | 4.5 |

### EXAMPLE 14: LOW LEVEL CROSSLINKING OF A SOLUBLE POLYMER TO OBTAIN A HIGH MOLECULAR WEIGHT SOLUBLE POLYMER; PREPARATION OF 0.75 MOL% CROSSLINKED SOLUBLE POLY(ALLYLAMINE)HCL

Poly(allylamine) Hydrochloride (200 g of 50% aqueous solution, 1.07 mol monomer equivalents) was dissolved a mixture of ethanol (213 mL) and water (125 mL) in a 1-liter, round-bottomed flask equipped with an overhead mechanical stirrer. The pH of the solution was brought to 10.0 - 10.2 by the addition of NaOH (50% solution). Epichlorohydrin (743 mg, 8.03 mmol) was then added to the rapidly stirred solution in one portion at room temperature (19-22°C). This mixture was stirred at room temperature (19-22°C) for 20 hours. After the 20 hour reaction time had elapsed, the pH was adjusted to 11.5 - 12.0 by the addition of a 50% NaOH solution. The reaction mixture was then poured into a 5-liter beaker containing 2 liters of methanol stirred with an overhead mechanical stirrer. A fine precipitate was observed as this mixture was stirred for 30 minutes. The mixture was vacuum filtered through Whatman 541 filter paper, and the clear filtrate was acidified with concentrated HCl (pH <1) producing a thick polymer precipitate and a cloudy solution. The cloudy methanol solution was decanted away from the crude solid product. The precipitate was dissolved in a minimum amount of water (approx. 300 mL) and acidified with concentrated HCl to a pH of < 2. The aqueous polymer solution was then poured with overhead mechanical stirring into a 3-liter beaker containing at least 5 volumes (approx. 1.5 liters) of methanol (Isopropanol can be used in place of methanol in this step). The polymeric product precipitated as a white solid. After stirring for 15 minutes, the precipitate was separated from solution by decantation and suspended in 2 liters of isopropyl alcohol. The solid was broken up using a metal spatula and the mixture was stirred for 2 hours. The isopropyl alcohol was then removed by decanting and the product was again suspended in 2 liters of fresh isopropyl alcohol. After 2 hours of stirring, the solvent was decanted away and the solid product was placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 88%.

### EXAMPLE 15: PREPARATION OF 0.75 MOL% CROSSLINKED POLY(DIALLYLAMINE)HCL

Poly(diallylamine) Hydrochloride (3250 g of 20% aqueous solution, 4.86 mol monomer equivalents) was placed in a 20-liter bucket equipped with an overhead mechanical stirrer. The pH of the solution was brought to 10.6 by the addition of NaOH (50% solution). Epichlorohydrin (2.86 mL, 0.037 mol) was then added to the rapidly stirred solution in one portion at room temperature (19-22°C). This mixture was stirred at room temperature (19-22°C) for 20 hours. A viscous solution resulted. Methanol (10 liters) was added, and the pH was adjusted to >11.5 using a 50% NaOH solution. This solution was then filtered to remove insoluble crosslinked polymer. The clear filtrate was acidified with concentrated HCl to a pH of <2, and the polymer product was precipitated with the addition of a large volume of ethanol. The solid was collected by decantation and washed with isopropanol. The solid product was then placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 355 g

### EXAMPLE 16: ALKYLATION OF AN INSOLUBLE GEL PREPARATION OF 10 MOL% DODECYL SUBSTITUTED, 3 MOL% CROSSLINKED POLY(ALLYLAMINE)HCL

Epichlorohydrin (3 mol%) crosslinked poly(allylamine)HCl (100 g of dry solid, 1.05 mol monomer equivalents) was suspended in methanol (1250 mL) in a 3-liter, round-bottomed flask equipped with an overhead mechanical stirrer, a condenser, and a thermocouple probe. Deionized water (750 mL) was slowly added to the suspension with good stirring, and the mixture was stirred until a uniform suspension resulted (approx. 3 hours). The mixture was then heated to 70°C, and the pH of the solution was brought to 10.0 - 10.2 by the addition of NaOH (50% solution). 1-Bromododecane (26.17 g, 0.105 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20 hour reaction time had elapsed, the mixture was cooled to room temperature and the pH was adjusted to < 1 using concentrated HCl. The reaction mixture was then poured into a 5-liter beaker containing 3 liters of methanol stirred with an overhead mechanical stirrer. The mixture was stirred until a uniform suspension resulted. The mixture was then vacuum filtered through Whatman 541 filter paper. The filtered polymer gel was collected and suspended in 3 liters of fresh methanol. The methanol suspension was acidified to a pH of < 1 with concentrated HCl. The mixture was then vacuum filtered through Whatman 541 filter paper. The filtered polymer gel was collected and suspended in 4-liters of 2M aqueous NaCl. The aqueous suspension was acidified to a pH of < 1 with concentrated HCl. The mixture was then vacuum filtered through Whatman 541 filter paper. The filtered polymer gel was then collected and purified by suspension into 4 liters of deionized water followed by vacuum filtration through Whatman 541 filter paper. The procedure of suspension into deionized water followed by vacuum filtration was repeated several times until the conductivity of the suspended polymer gel was < 0.5 mS/cm. After the final vacuum filtration, the polymer gel was transferred into several Pyrex drying trays and placed into a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 82%.

**Table 2.**

| **Alkylation Reactions of Xlinked Polyamines Using the Procedure in Example 16** | | | |
|---|---|---|---|
| **Example** | **Polyamine** | **Alkylating Agent** | **mol%** |
| 17 | Poly(allylamine)HCl Xlink 3 mol% | 1-Bromohexane | 5 |
| 18 | Poly(allylamine)HCl Xlink 3 mol% | 1-Bromohexane | 10 |
| 19 | Poly(allylamine)HCl Xlink 3 mol% | 1-Bromohexane | 25 |
| 20 | Poly(allylamine)HCl Xlink 3 mol% | 1-Bromohexane | 50 |
| 21 | Poly(allylamine)HCl Xlink 3 mol% | 1-Bromooctane | 5 |
| 22 | Poly(allylamine)HCl Xlink 3 mol% | 1-Bromooctane | 10 |
| 23 | Poly(allylamine)HCI Xlink 3 mol% | 1-Bromooctane | 25 |
| 24 | Poly(allylamine)HCl Xlink 3 mol% | 1-Bromooctane | 50 |
| 25 | Poly(allylamine)HCl Xlink 3 mol% | 1-Bromododecane | 5 |
| 26 | Poly(allylamine)HCl Xlink 3 mol% | 1-Bromododecane | 25 |
| 27 | Poly(allylamine)HCl Xlink 3 mol% | 1-Bromododecane | 50 |
| 28 | Poly(allylamine)HCl Xlink 3 mol% | 1-Bromooctadecane | 5 |
| 29 | Poly(allylamine)HCl Xlink 3 mol% | 1-Bromooctadecane | 10 |
| 30 | Poly(allylamine)HCl Xlink 3 mol% | 1-Bromooctadecane | 25 |
| 31 | Poly(allylamine)HCl Xlink 3 mol% | 1-Bromo-2-ethylhexane | 5 |
| 32 | Poly(allylamine)HCl Xlink 3 mol% | 1-Bromo-2-ethylhexane | 10 |
| 33 | Poly(allylamine)HCl Xlink 3 mol% | 1-Bromo-2-ethylhexane | 25 |
| 34 | Poly(allylamine)HCl Xlink 3 mol% | 1-Bromo-2-ethylhexane | 50 |
| 35 | Poly(allylamine)HCl Xlink 3 mol% | (Bromomethyl)cyclohexane | 5 |
| 36 | Poly(allylamine)HCl Xlink 3 mol% | (Bromomethyl)cyclohexane | 10 |
| 37 | Poly(allylamine)HCl Xlink 3 mol% | (Bromomethyl)cyclohexane | 25 |
| 38 | Poly(allylamine)HCl Xlink 3 mol% | (3-Bromopropyl)trimethylammonium Bromide | 5 |
| 39 | Poly(allylamine)HCl Xlink 3 mol% | (3-Bromopropyl)trimethylammonium Bromide | 10 |
| 40 | Poly(allylamine)HCl Xlink 3 mol% | (3-Bromopropyl)trimethylammonium Bromide | 25 |
| 41 | Poly(allylamine)HCl Xlink 3 mol% | (6-Bromohexyl)trimethylammonium Chloride | 10 |
| 42 | Poly(allylamine)HCl Xlink 3 mol% | 1,3-Propane Sultone | 5 |
| 43 | Poly(allylamine)HCl Xlink 3 mol% | 1,3-Propane Sultone | 10 |
| 44 | Poly(allylamine)HCl Xlink 3 mol% | 1,3-Propane Sultone | 25 |
| 45 | Poly(allylamine)HCl Xlink 3 mol% | 1-Bromoacetic Acid | 5 |
| 46 | Poly(allylamine)HCl Xlink 3 mol% | 1-Bromoacetic Acid | 10 |
| 47 | Poly(allylamine)HCl Xlink 3 mol% | 1-Bromoacetic Acid | 25 |
| 48 | Poly(allylamine)HCl Xlink 3 mol% | 2-Bromoethanesulfonic Acid Na salt | 5 |
| 49 | Poly(allylamine)HCl Xlink 3 mol% | 2-Bromoethanesulfonic Acid Na salt | 10 |
| 50 | Poly(allylamine)HCl Xlink 3 mol% | 2-Bromoethanesulfonic Acid Na salt | 25 |
| 51 | Poly(allylamine)HCl Xlink 3 mol% | 2-Bromododecane (6-Bromohexyl) trimethylammonium Chloride | 10 10 |
| 52 | Poly(allylamine)HCl Xlink 6 mol% | 2-Bromododecane | 10 |
| 53 | Poly(allylamine)HCl Xlink 6 mol% | 2-Bromododecane (6-Bromohexyl) trimethylammonium Chloride | 10 10 |
| 54 | Poly(allylamine)HCl Xlink 3 mol% | (4-chlorobutyl) dodecyldimethylammonium Bromide | 10 |
| 55 | Poly(allylamine)HCl Xlink 3 mol% | (4-chlorobutyl) dodecyldimethylammonium Bromide | 40 |
| 56 | Poly(ethylenimine) Xlink 3 mol% | 1-Bromohexane | 5 |
| 57 | Poly(ethylenimine) Xlink 3 mol% | 1-Bromohexane | 10 |
| 58 | Poly(ethylenimine) Xlink 3 mol% | 1-Bromohexane | 25 |
| 59 | Poly(ethylenimine) Xlink 3 mol% | 1-Bromooctane | 5 |
| 60 | Poly(ethylenimine) Xlink 3 mol% | 1-Bromooctane | 10 |
| 61 | Poly(ethylenimine) Xlink 3 mol% | 1-Bromooctane | 25 |
| 62 | Poly(ethylenimine) Xlink 3 mol% | 1-Bromododecane | 5 |
| 63 | Poly(ethylenimine) Xlink 3 mol% | 1-Bromododecane | 10 |
| 64 | Poly(ethylenimine) Xlink 3 mol% | 1-Bromododecane | 25 |
| 65 | Poly(diallylamine)HCl Xlink 4.5 mol% | 1-Bromohexane | 5 |
| 66 | Poly(diallylamine)HCl Xlink 4.5 mol% | 1-Bromohexane | 25 |
| 67 | Poly(diallylamine)HCl Xlink 4.5 mol% | 1-Bromohexane | 50 |
| 68 | Poly(diallylamine)HCl Xlink 4.5 mol% | 1-Bromooctane | 5 |
| 69 | Poly(diallylamine)HCl Xlink 4.5 mol% | 1-Bromooctane | 30 |
| 70 | Poly(diallylamine)HCl Xlink 4.5 mol% | 1-Bromooctane | 40 |
| 71 | Poly(diallylamine)HCl Xlink 4.5 mol% | 1-Bromododecane | 5 |
| 72 | Poly(diallylamine)HCl Xlink 4.5 mol% | 1-Bromododecane | 11 |
| 73 | Poly(diallylamine)HCl Xlink 4.5 mol% | 1-Bromododecane | 25 |
| 74 | Poly(diallylamine)HCl Xlink 4.5 mol% | (4-chlorobutyl) dodecyldimethylammonium Bromide | 10 |
| 75 | Poly(diallylamine)HCl Xlink 4.5 mol% | (4-chlorobutyl) dodecyldimethylammonium Bromide | 20 |
| 76 | Poly(diallylamine)HCl Xlink 4.5 mol% | (4-chlorobutyl) dodecyldimethylammonium Bromide | 30 |

### EXAMPLE 77: ACETYLATION OF A CROSSLINKED POLYMER GEL PREPARATION OF 25 MOL% ACETYLATED-POLY(ALLYLAMINE)HCL

Epichlorohydrin (3 mol%) crosslinked poly(allylamine)HCl (57.4 g of dry solid, 0.602 mol monomer equivalents) was suspended in methanol (1 liter) in a 2-liter, round- bottomed flask equipped with an overhead mechanical stirrer, a condenser, and a thermocouple probe. Deionized water (550 mL) was slowly added to the suspension with good stirring, and the mixture was stirred until a uniform suspension resulted (approx. 3 hours). The mixture was then cooled to 15°C with an ice bath, and the pH of the solution was brought to 9.5 by the addition of NaOH (50% solution). Acetic anhydride (15.41 g, 0.151 mol) was then added to the stirred solution in one portion. This mixture was stirred at 15°C for 30 minutes. The solution pH was maintained at 9.5 during this time by the addition of small quantities of 50% NaOH. After the 30 minutes it was observed that the pH of the mixture was stable. The crude polymer product was then purified by suspension into 4 liters of deionized water followed by vacuum filtration through Whatman 541 filter paper. The procedure of suspension into deionized water followed by vacuum filtration was repeated several times until the conductivity of the suspended polymer gel was < 1 mS/cm. The polymer gel was then suspended in deionized water (2 liters) and the mixture was acidified with concentrated HCl to a pH of <2.5. The mixture was then filtered and transferred into several Pyrex drying trays. The trays were placed into a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 49.9 g

**Table 3.**

| **Acetylation reactions according to the procedure of Example 77** | | |
|---|---|---|
| Example | Polyamine | mol% Acetylation |
| 78 | Poly(allylamine) HCl | 50 |
| 79 | Poly(diallylamine) HCl | 25 |
| 80 | Poly(diallylamine) HCl | 50 |
| 81 | Poly(diallylamine) HCl | 100 |

### EXAMPLE 82: ALKYLATION OF A SOLUBLE POLYMER PREPARATION OF 10 MOL% DODECYL-POLY(ALLYLAMINE)HCL

Poly(allylamine) Hydrochloride (200 g of 50% aqueous solution, 1.07 mol monomer equivalents) was dissolved a mixture of ethanol (213 mL) and water (125 mL), and was heated to 70°C in a 1-liter, round-bottomed flask equipped with an overhead mechanical stirrer, a condenser, and a thermocouple probe. The pH of the solution was brought to 10.0 - 10.2 by the addition of NaOH (50% solution). 1-Bromododecane (26.66 g, 0.107 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20 hour reaction time had elapsed, the mixture was cooled to room temperature and the pH was adjusted to 11.5 - 12.0 by the addition of a 50% NaOH solution. The reaction mixture was then poured into a 5-liter beaker containing 2 liters of methanol stirred with an overhead mechanical stirrer. A fine precipitate was observed as this mixture was stirred for 30 minutes. The mixture was vacuum filtered through Whatman 541 filter paper, and the clear filtrate was acidified with concentrated HCl (pH <1) producing a thick polymer precipitate and a cloudy solution. The cloudy methanol solution was decanted away from the crude solid product. The precipitate was dissolved in a minimum amount of water (approx. 300 mL) and acidified with concentrated HCl to a pH of < 2. The aqueous polymer solution was then poured with overhead mechanical stirring into a 3-liter beaker containing at least 5 volumes (approx. 1.5 liters) of methanol (isopropanol can be used in place of methanol in this step). The polymeric product precipitated as a white solid. After stirring for 15 minutes, the precipitate was separated from solution by decantation and suspended in 2 liters of isopropyl alcohol. The solid was broken up using a metal spatula and the mixture was stirred for 2 hours. The isopropyl alcohol was then removed by decanting and the product was again suspended in 2 liters of fresh isopropyl alcohol. After 2 hours of stirring, the solvent was decanted away and the solid product was placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 86%.

### EXAMPLE 83: PREPARATION OF 0.75 MOL% CROSSLINKED, 10 MOL% HEXYL-POLY(ALLYLAMINE) HCL

The procedure of Example 82 was used. The 50% aqueous solution of poly(allylamine)HCl was replaced with an equivalent amount of a 50% aqueous solution of the polymer product of Example 14. In place of the 1-bromododecane, 1-bromohexane (17.66 g, 0.107 mol) was used.

### EXAMPLE 84: PREPARATION OF 0.75 MOL% CROSSLINKED, 10 MOL% DODECYL-POLY(ALLYLAMINE) HCL

The procedure of Example 82 was used. The 50% aqueous solution of poly(allylamine)HCl was replaced with an equivalent amount of a 50% aqueous solution of the polymer product of Example 14.

### EXAMPLE 85: PREPARATION OF 0.75 MOL% CROSSLINKED, 2 MOL% OCTADECYL-POLY(ALLYLAMINE) HCL

The procedure of Example 82 was used. The 50% aqueous solution of poly(allylamine)HCl was replaced with an equivalent amount of a 50% aqueous solution of the polymer product of Example 14. In place of the 1-bromododecane, 1-bromooctadecane (7.13 g, 0.021 mol) was used.

### EXAMPLE 86: PREPARATION OF 0.75 MOL% CROSSLINKED, 25 MOL% DODECYL-POLY(ALLYLAMINE)HCL

The procedure of Example 82 was used. The amount of dodecyl bromide used was 67.3 g, 0.27 mol. The 50% aqueous solution of poly(allylamine)HCl was replaced with an equivalent amount of a 50% aqueous solution of the polymer product of Example 14.

### EXAMPLE 87: PREPARATION OF 25 MOL% HEXYL-POLY(ALLYLAMINE)HCL 0.75 MOL% EPICHLOROHYDRIN CROSSLINKED

Poly(allylamine) Hydrochloride 0.75% epichlorohydrin crosslinked from Example 14 (200 g of 50% aqueous solution, 1.07 mol monomer equivalents) was dissolved in a mixture of ethanol (213 mL) and water (125 mL), and was heated to 70°C in a 1-liter, round-bottomed flask equipped with an overhead mechanical stirrer, a condenser, and a thermocouple probe. The pH of the solution was brought to 10.0 - 10.2 by the addition of NaOH (50% solution). 1-Bromohexane (44.2 g, 0.27 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20 hour reaction time had elapsed, the mixture was cooled to room temperature and the pH was adjusted to 11.5 - 12.0 by the addition of a 50% NaOH solution. The reaction mixture was then poured into a 5-liter beaker containing 2 liters of methanol stirred with an overhead mechanical stirrer. In this case, no precipitate was formed. The methanol solution was evaporated to dryness giving a stick solid. The solid was dissolved in 800 mL of methanol and 3 liters of hexane was added to precipitate the polymer. After collection by filtration, the polymeric solid was washed with an additional 1 liter of hexane and collected by filtration. The solid product was placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 89.3 g

### EXAMPLE 88: PREPARATION OF 50 MOL% HEXYL-POLY(ALLYLAMINE)HCL 0.75 MOL% EPICHLOROHYDRIN CROSSLINKED

Poly(allylamine) Hydrochloride 0.75% epichlorohydrin crosslinked from Example 14 (200 g of 50% aqueous solution, 1.07 mol monomer equivalents) was dissolved in a mixture of ethanol (213 mL) and water (125 mL), and was heated to 70°C in a 1-liter, round-bottomed flask equipped with an overhead mechanical stirrer, a condenser, and a thermocouple probe. The pH of the solution was brought to 10.0 - 10.2 by the addition of NaOH (50% solution). 1-Bromohexane (88.4 g, 0.51 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20 hour reaction time had elapsed, the mixture was cooled to room temperature giving a white suspension of polymer. The white solid was allowed to settle, and the solution was decanted away. Deionized water (1.5 liters) was added and the slurry was stirred for 15 minutes. The solid was allowed to settle and the solution was removed by decantation. The solid was then dissolved in 800 mL of isopropanol. Concentrated HCI (120 mL) was added but no precipitate was seen. Hexane (3 liters) was added and a white solid precipitated from solution. After collection by filtration. the polymeric solid was washed with an additional 1.5 liters of hexane and collected by filtration. The solid product was placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 117 g

### EXAMPLE 89: PREPARATION OF 5 MOL% DODECYL-POLY(ALLYLAMINE)HCL 0.75 MOL% EPICHLOROHYDRIN CROSSLINKED

Poly(allylamine) Hydrochloride 0.75% epichlorohydrin crosslinked from Example 14 (200 g of 50% aqueous solution, 1.07 mol monomer equivalents) was dissolved in a mixture of ethanol (213 mL) and water (125 mL), and was heated to 70°C in a 1-liter, round-bottomed flask equipped with an overhead mechanical stirrer, a condenser, and a thermocouple probe. The pH of the solution was brought to 10.0 - 10.2 by the addition of NaOH (50% solution). 1-Bromododecane (13.33 g, 0.054 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20 hour reaction time had elapsed, the mixture was cooled to room temperature and the pH was adjusted to 11.5 - 12.0 by the addition of a 50% NaOH solution. The reaction mixture was then poured into a 5-liter beaker containing 2 liters of methanol stirred with an overhead mechanical stirrer. A fine precipitate was observed as this mixture was stirred for 30 minutes. The mixture was vacuum filtered through Whatman 541 filter paper, and the clear filtrate was acidified with concentrated HCl (pH <1) producing a thick polymer precipitate and a cloudy solution. The cloudy methanol solution was decanted away from the crude solid product. Isopropanol (2.5 liters) was added and the solid was broken into small pieces with a spatula. The solid was collected by decantation and washed a second time with fresh isopropanol. The solid product was placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 81 g

### EXAMPLE 90: PREPARATION OF 5 MOL% OCTADECYL-POLY(ALLYLAMINE)HCL 0.75 MOL% EPICHLOROHYDRIN CROSSLINKED

Poly(allylamine) Hydrochloride 0.75% epichlorohydrin crosslinked from Example 14 (200 g of 50% aqueous solution, 1.07 mol monomer equivalents) was dissolved a mixture of ethanol (213 mL) and water (125 mL), and was heated to 70°C in a 1-liter, round-bottomed flask equipped with an overhead mechanical stirrer, a condenser, and a thermocouple probe. The pH of the solution was brought to 10.0 - 10.2 by the addition of NaOH (50% solution). 1-Bromooctadecane (17.84 g, 0.054 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20 hour reaction time had elapsed, the mixture was cooled to room temperature and poured into a.5-liter beaker containing 2 liters of methanol stirred with an overhead mechanical stirrer. The mixture was acidified with concentrated HCl (pH <1) producing a thick polymer precipitate. The methanol solution was decanted away from the crude solid product, and water (approx. 300 mL) was added to disperse the polymeric product into a slurry. Methanol (900 mL) was added giving a dense precipitate. The solution was decanted away and the polymer was slurried in water a second time and precipitated with methanol. The solid polymer was then washed with 2.5 liters of isopropanol, and then with 1 liter of diethyl ether. The solid product was then placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 111 g

### EXAMPLE 91: PREPARATION OF 10 MOL% OCTADECYL-POLY(ALLYLAMINE)HCL 0.75 MOL% EPICHLOROHYDRIN CROSSLINKED

The procedure of Example 90 was used. The amount of octadecyl bromide used was 35.67 g, 0.107 mol. Yield = 124 g

### EXAMPLE 92: PREPARATION OF 5 MOL% DOCOSYL-POLY(ALLYLAMINE)HCL 0.75 MOL% EPICHLOROHYDRIN CROSSLINKED

The procedure of Example 90 was used. The amount of docosyl bromide used was 21.03 g, 0.054 mol. Yield = 96 g

### EXAMPLE 93: PREPARATION OF 10 MOL% DOCOSYL-POLY(ALLYLAMINE)HCL 0.75 MOL% EPICHLOROHYDRIN CROSSLINKED

The procedure of Example 90 was used. The amount of docosyl bromide used was 41.68 g, 0.107 mol. Yield = 101 g

### EXAMPLE 94: PREPARATION OF 25 MOL% HEXYL-POLYETHYLENIMINE HCL

Polyethylenimine (200 g of a 50% aqueous solution from Aldrich Chemical Co., 2.32 mol monomer equivalents) was dissolved a mixture of ethanol (213 mL) and water (125 mL), and was heated to 70°C in a 1-liter, round-bottomed flask equipped with an overhead mechanical stirrer, a condenser, and a thermocouple probe. 1-Bromohexane (95.7 g, 0.58 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20 hour reaction time had elapsed, the mixture was cooled to room temperature and poured into a 20-liter bucket containing 3 liters of methanol stirred with an overhead mechanical stirrer. The mixture was acidified with concentrated HCI (pH <2), but no precipitate was formed. Isopropanol (6 liters) was added, giving a small amount of precipitate. Diethyl ether was then added (3 liters) and the crude product precipitated. The solvent was decanted away from the product. The crude product was then redispersed in 750 mL of deionized water. The pH was adjusted to <2 using concentrated HCI. Acetonitrile (5 liters) was then added to precipitate the polymer. The solid was collected by decantation and washed with 2 liters of isopropanol. The solid product was then placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 172 g

### EXAMPLE 95: PREPARATION OF 50 MOL% HEXYL-POLYETHYLENIMINE HCL

Polyethylenimine (200 g of a 50% aqueous solution from Aldrich Chemical Co., 2.32 mol monomer equivalents) was dissolved in a mixture of ethanol (213 mL) and water (125 mL), and was heated to 70°C in a 1-liter, round-bottomed flask equipped with an overhead mechanical stirrer, a condenser, and a thermocouple probe. 1-Bromohexane (191.5 g, 1.16 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20-hour reaction time had elapsed, the mixture was cooled to room temperature and poured into a 20-liter bucket containing 2 liters of methanol stirred with an overhead mechanical stirrer. The mixture was acidified with concentrated HCl (pH <2), but no precipitate was formed. Diethyl ether was then added (3 liters) and the crude product precipitated. The solvent was decanted away from the product. The crude product was then redispersed in ethanol (3 liters). The pH was adjusted to >11.5 using concentrated NaOH. The free base polymer dissolved, leaving a suspension of salts. The mixture was vacuum filtered through Whatman 541 filter paper, and the clear filtrate was acidified with concentrated HCl (pH <1). Diethyl ether was then added to precipitate the product, which was then collected by decantation. The solid product was then placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 110 g

### EXAMPLE 96: PREPARATION OF 5 MOL% DODECYL-POLYETHYLENIMINE HCL

Polyethylenimine (200 g of a 50% aqueous solution from Aldrich Chemical Co., 2.32 mol monomer equivalents) was dissolved in a mixture of ethanol (213 mL) and water (125 mL), and was heated to 70°C in a 1-liter, round-bottomed flask equipped with an overhead mechanical stirrer, a condenser, and a thermocouple probe. 1-Bromododecane (28.9 g, 0.116 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20 hour reaction time had elapsed, the mixture was cooled to room temperature and poured into a 20-liter bucket containing 2 liters of methanol stirred with an overhead mechanical stirrer. The mixture was acidified with concentrated HCl (pH <2), resulting in the precipitation of some polymer. Isopropanol was then added (3 liters) and the crude product precipitated. The solvent was decanted away from the product. The crude product was then redispersed in water (750 mL) and methanol (400 mL) and the pH was adjusted to <2 using concentrated HCI. Isopropanol (6 liters) was then added to precipitate the product, which was collected by decantation. The solid product was then placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 146 g

### EXAMPLE 97: PREPARATION OF 10 MOL% DODECYL-POLYETHYLENIMINE HCL

Polyethylenimine (200 g of a 50% aqueous solution from Aldrich Chemical Co., 2.32 mol monomer equivalents) was dissolved in a mixture of ethanol (213 mL) and water (125 mL), and was heated to 70°C in a 1-liter, round-bottomed flask equipped with an overhead mechanical stirrer, a condenser, and a thermocouple probe. 1-Bromododecane (57.8 g, 0.232 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20 hour reaction time had elapsed, the mixture was cooled to room temperature and poured into a 20-liter bucket containing 2 hters of methanol stirred with an overhead mechanical stirrer. The mixture was acidified with concentrated HCl (pH <2), resulting in the precipitation of some polymer. Isopropanol was then added (3 liters) and the crude product precipitated. The solvent was decanted away from the product. The crude product was then redispersed in water (750 mL) and methanol (400 mL) and the pH was adjusted to <2 using concentrated HCl. Isopropanol (6 liters) was then added to precipitate the product, which was collected by decantation. The solid product was then placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 119 g

### EXAMPLE 98: PREPARATION OF 25 MOL% DODECYL-POLYETHYLENIMINE HCL

Polyethylenimine (200 g of a 50% aqueous solution from Aldrich Chemical Co., 2.32 mol monomer equivalents) was dissolved in a mixture of ethanol (213 mL) and water (125 mL), and was heated to 70°C in a 1-liter, round-bottomed flask equipped with an overhead mechanical stirrer, a condenser, and a thermocouple probe. 1-Bromododecane (144.6 g, 0.58 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20 hour reaction time had elapsed, the mixture was cooled to room temperature and poured into a 20-liter pail containing 5 liters of ethanol stirred with an overhead mechanical stirrer. The mixture was acidified with concentrated HCl (pH <2), resulting in the precipitation of the crude product. The solvent was decanted, and the crude product was then redissolved in water (750 mL). The pH was adjusted to <2 using concentrated HCl. Isopropanol (6 liters) was then added to precipitate the product, which was collected by decantation. The solid product was then placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 197 g

### EXAMPLE 99: PREPARATION OF 2.5 MOL% OCTADECYL-POLYETHYLENIMINE HCL

Polyethylenimine (200 g of a 50% aqueous solution from Aldrich Chemical Co., 2.32 mol monomer equivalents) was dissolved in a mixture of ethanol (213 mL) and water (125 mL), and was heated to 70°C in a 1-liter, round-bottomed flask equipped with an overhead mechanical stirrer, a condenser, and a thermocouple probe. 1-bromooctadecane (19.3 g, 0.058 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20 hour reaction time had elapsed, the mixture was cooled to room temperature and poured into a 20-liter bucket containing 3.5 liters of methanol stirred with an overhead mechanical stirrer. The mixture was acidified with concentrated HCl (pH <2), resulting in the precipitation ofthe crude product. The solvent was decanted, and the crude product was then redissolved in water (1100 mL). The pH was adjusted to <2 using concentrated HCl. Isopropanol (6 liters) was then added to precipitate the product, which was collected by decantation. The solid was washed with another 2 liters of clean isopropanol and collected by decantation. The solid product was then placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 169 g

### EXAMPLE 100: PREPARATION OF 5 MOL% OCTADECYL-POLYETHYLENIMINE HCL

The procedure of Example 99 was used. The amount of 1-bromooctadecane used 38.7 g, 0.116 mol. Yield = 198 g

### EXAMPLE 101: PREPARATION OF 2 MOL% DOCOSYL-POLYETHYLENIMINE HCL

Polyethylenimine (200 g of a 50% aqueous solution from Aldrich Chemical Co., 2.32 mol monomer equivalents) was dissolved in a mixture of ethanol (213 mL) and water (125 mL), and was heated to 70°C in a 1-liter, round-bottomed flask equipped with an overhead mechanical stirrer, a condenser, and a thermocouple probe. 1-Bromodocosane (18.1 g, 0.046 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20 hour reaction time had elapsed, the mixture was cooled to room temperature and poured into a 20-liter bucket containing 2.4 liters of ethanol stirred with an overhead mechanical stirrer. The mixture was acidified with concentrated HCl (pH <2), resulting in the precipitation the crude product. The solvent was decanted, and the crude product was then redissolved in water (700 mL). The pH was adjusted to <2 using concentrated HCl. Isopropanol (6 liters) was then added to precipitate the product, which was collected by decantation. The solid was washed with another 2 liters of clean isopropanol and collected by decantation. The solid product was then placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 154 g

### EXAMPLE 102: PREPARATION OF 5 MOL% DOCOSYL-POLYETHYLENIMINE HCL

The procedure of Example 101 was used. The amount of 1-bromodocosane used was 45.2 g, 0.116 mol. Yield = 160 g

### EXAMPLE 103: PREPARATION OF 5 MOL% DODECYL-POLY(DIALLYLAMINE)HCL 0.75 MOL% EPICHLOROHYDRIN CROSSLINKED

Poly(diallylamine) Hydrochloride 0.75% epichlorohydrin crosslinked from Example 15 (60 g, 0.45 mol monomer equivalents) was dispersed in ethanol (500 mL) in a 1-liter, round-bottomed flask equipped with an overhead mechanical stirrer, a condenser, and a thermocouple probe. NaOH was added (30 g of a 50% solution), along with deionized water (200 mL) and the mixture was heated to 70°C. The pH of the solution was brought to 10.0 - 10.2 by the addition of NaOH (50% solution). 1-Bromododecane (5.6 g, 0.023 mol) was then added to the stirred solution in one portion. This mixture was stirred at 70°C for 20 hours. The solution pH was checked periodically during this time, and was maintained at 10.0 - 10.2 by the addition of small quantities of 50% NaOH. After the 20 hour reaction time had elapsed, the mixture was cooled to room temperature and poured into a 5-liter beaker containing 3 liters of deionized water stirred with an overhead mechanical stirrer. The crude polymeric product precipitated from solution and was collected by decantation. The crude product was added to a mixture of 300 mL deionized water and 300 mL ethanol. The mixture was acidified with concentrated HCl (pH <1) and stirred for at least 2 hours. Isopropanol (3 liters) was then added to precipitate the product. The solid polymer was then washed with clean isopropanol (2 liters). The solid product was then placed in a convection oven at 70°C to dry (24 - 48 hours). The dried solid was ground to a fine powder using a lab mill with stainless steel blades, and was passed through a sieve (50 mesh) to remove large granules. The ground product was then placed in a vacuum oven at 60°C and 28 mmHg for at least 16 hours. Yield = 27 g

### EXAMPLE 104: PREPARATION OF 10 MOL% DODECYL-POLY(DIALLYLAMINE)HCL 0.75 MOL% EPICHLOROHYDRIN CROSSLINKED

The procedure of Example 104 was used. The amount of 1-bromododecane used was 11.2 g, 0.045 mol. Yield = 46 g

### EXAMPLE 105: PREPARATION OF 25 MOL% DODECYL-POLY(DIALLYLAMINE)HCL 0.75 MOL% EPICHLOROHYDRIN CROSSLINKED

The procedure of Example 104 was used. The amount of 1-bromododecane used was 28.2 g, 0.113 mol. Yield = 52 g

### EXAMPLE 106: PREPARATION OF 5 MOL% HEXYL-POLY(DIALLYLAMINE)HCL 0.75 MOL% EPICHLOROHYDRIN CROSSLINKED

The procedure of Example 104 was used. The amount of 1-bromohexane used was 3.72 g, 0.023 mol.

### EXAMPLE 107: PREPARATION OF 10 MOL% HEXYL-POLY(DIALLYLAMINE)HCL 0.75 MOL% EPICHLOROHYDRIN CROSSLINKED

The procedure of Example 104 was used. The amount of 1-bromohexane used was 7.43 g, 0.045 mol. Yield = 36 g

### EXAMPLE 108: PREPARATION OF 25 MOL% HEXYL-POLY(DIALLYLAMINE)HCL 0.75 MOL% EPICHLOROHYDRIN CROSSLINKED

The procedure of Example 104 was used. The amount of 1-bromohexane used was 18.65 g, 0.113 mol. Yield = 49 g

### EXAMPLE 109: PREPARATION OF 50 MOL% HEXYL-POLY(DIALLYLAMINE)HCL 0.75 MOL% EPICHLOROHYDRIN CROSSLINKED

The procedure of Example 104 was used. The amount of 1-bromohexane used was 38.0 g, 0.230 mol. Yield = 67 g

### EXAMPLE 110: PREPARATION OF 2 MOL% OCTADECYL-POLY(DIALLYLAMINE)HCL 0.75 MOL% EPICHLOROHYDRIN CROSSLINKED

The procedure of Example 104 was used. The amount of 1-bromooctadecane used was 3.0 g, 0.009 mol. Yield = 23 g

### EXAMPLE 111: PREPARATION OF 5 MOL% OCTADECYL-POLY(DIALLYLAMINE)HCL 0.75 MOL% EPICHLOROHYDRIN CROSSLINKED

The procedure of Example 104 was used. The amount of 1-bromooctadecane used was 7.7 g, 0.023 mol. Yield = 35 g

### EXAMPLE 112: PREPARATION OF 10 MOL% OCTADECYL-POLY(DIALLYLAMINE)HCL 0.75 MOL% EPICHLOROHYDRIN CROSSLINKED

The procedure of Example 104 was used. The amount of 1-bromooctadecane used was 15.0 g, 0.045 mol. Yield = 35 g

### EXAMPLE 113: PREPARATION OF COPOLYMER OF ACRYLAMIDE (20 MOLE %)/TRIMETHYLAMINOETHYL ACRYLCHLORIDE Q SALT (TMAEAC) ( 78 MOLE%)/OCTADECYLACRYLATE (2 MOLE %)

To a 1-liter, three-necked flask equipped with condenser and stir bar, were added trimethylaminoethyl acrylchloride quaternary salt (TMAEAC) 50% aqueous solution (150.90 g of 50% solution, 390 mmoles, 78 mole%), acrylamide (7.11 g, 100 mmoles, 20 mole%), octadecyl acrylate ( 3.25 g, 10 mmoles, 2 mole%) and isopropanol (400 mL). The mixture was purged with nitrogen for 10 min before the addition of a radical initiator, AIBN ( 330 mg, 2 mmoles). The mixture was heated to 70°C for 16 hours. At the end of the 16 hour reaction time, the reaction mixture was allowed to cool to room temperature and poured into a beaker containing isopropanol ( 1 liter). The polymer was precipitated out as a white solid, which was collected and ground to small pieces in a blender using isopropanol as a solvent. The pieces were collected by filtration and the polymer was dried under vacuum at 60°C for 2 days. The material was ground to a fine powder (82 g), which was used for the *in vitro* and *in vivo* studies.

The following polymers of Table 4 with varying composition of acrylamide, TMAEAC and octadecyl acrylate were prepared using the above procedure.

**Table 4**

| Example No. | Acrylamide (mole %) | TMAEAC (mole %) | Octadecyl acrylate (mole %) |
|---|---|---|---|
| 114 | 20 | 75 | 5 |
| 115 | 20 | 70 | 10 |
| 116 | 35 | 63 | 2 |
| 117 | 35 | 60 | 5 |
| 118 | 35 | 55 | 10 |
| 119 | 50 | 48 | 2 |
| 120 | 50 | 45 | 5 |
| 121 | 50 | 40 | 10 |
| 122 | 0 | 98 | 2 |
| 123 | 0 | 95 | 5 |
| 124 | 0 | 90 | 10 |

### EXAMPLE 125: PREPARATION OF COPOLYMER OF ACRYLAMIDE (20 MOLE %)/TRIMETHYLAMINOETHYL ACRYLCHLORIDE Q SALT (TMAEAC) ( 75 MOLE %)/DODECYLACRYLATE (5 MOLE %)

To a 1-liter, three-necked flask equipped with condenser and stir bar, were added trimethylaminoethyl acrylchloride quaternary salt (TMAEAC) 50% aqueous solution (145.80 g of 50 % solution, 375 mmoles, 75 mole%), acrylamide (7.11 g, 100 mmoles, 20 mole%), dodecyl acrylate (6.01 g, 25 mmoles, 5 mole%) and isopropanol (400 mL). The mixture was purged with nitrogen for 10 min before the addition of a radical initiator, AIBN ( 330 mg, 2 mmoles). The mixture was heated to 70° C for 16 hours. The reaction mixture was allowed to cool to room temperature and poured into a beaker containing isopropanol ( 1 liter). The polymer was precipitated out as a white solid, which was collected and ground to small pieces in a blender using isopropanol as a solvent. The pieces were collected by filtration and the polymer was dried under vacuum at 60°C for 2 days. The material was ground to a fine powder (80 g), which was used for the *in vitro* and *in vivo* studies. The polymers of Table 5 were prepared using the above procedure.

**Table 5**

| Example No. | Acrylamide (mole %) | TMAEAC (mole %) | Dodecylacrylate (mole %) |
|---|---|---|---|
| 126 | 20 | 70 | 10 |
| 127 | 20 | 55 | 25 |
| 128 | 35 | 60 | 5 |
| 129 | 35 | 55 | 10 |
| 130 | 35 | 40 | 25 |
| 131 | 50 | 45 | 5 |
| 132 | 50 | 40 | 10 |
| 133 | 50 | 25 | 25 |
| 134 | 0 | 95 | 5 |
| 135 | 0 | 90 | 10 |
| 136 | 0 | 75 | 25 |
| 137 | 0 | 98 | 2 |
| 138 | 20 | 78 | 2 |
| 139 | 35 | 63 | 2 |
| 140 | 50 | 48 | 2 |

### EXAMPLE 141: PREPARATION OF COPOLYMER OF ACRYLAMIDE (20 MOLE %)/TRIMETHYLAMINOETHYL ACRYLCHLORIDE Q SALT (TMAEAC) ( 80 MOLE %)

To a 1-liter, three-necked flask equipped with condenser and stir bar, were added trimethylaminoethyl acrylchloride quaternary salt (TMAEAC) 50% aqueous solution (154.76 g of 50% solution, 400 mmoles, 80 mole%), acrylamide (7.11g, 100 mmoles, 20 mole%), and isopropanol (400 mL). The mixture was purged with nitrogen for 10 min before the addition of a radical initiator, AIBN ( 330 mg, 2 mmoles). The mixture was heated to 70°C for 16 hours. At the end of reaction, reaction mixture was allowed to cool to room temperature and poured into a beaker containing isopropanol (1 liter). The polymer was precipitated out as a white solid, which was collected and ground to small pieces in a blender using isopropanol as a solvent. The pieces were collected by filtration and the polymer was dried under vacuum at 60°C for 2 days. The material was ground to a fine powder (80 g), which was used for the *in vitro* and *in vivo* studies.
The polymers of Table 6 were prepared using the above procedure

**Table 6**

| Example No | Acrylamide ( mole %) | TMAFAC (mole %) |
|---|---|---|
| 142 | 0 | 100 |
| 143 | 10 | 90 |
| 141 | 20 | 80 |
| 144 | 35 | 65 |
| 145 | 50 | 50 |

### EXAMPLE 146: PREPARATION OF METHYLENEBISACRYLAMIDE (4 MOLE %) CROSS-LINKED POLYMERS OF ACRYLAMIDE (20 MOLE %)/TRIMETHYLAMINOETHYL ACRYLCHLORIDE Q SALT (TMAEAC) (78 MOLE %)/ OCTADECYL ACRYLATE (2 MOLE %)

To a 1-liter, three-necked flask equipped with condenser and stir bar, were added trimethylaminoethyl acrylchloride quaternary salt (TMAEAC) 50% aqueous solution (150.90 g of 50% solution, 390 mmoles, 78 mole%), acrylamide (7.11 g , 100 mmoles, 20 mole%), octadecyl acrylate ( 3.25 g, 10 mmoles, 2 mole%), methylenebisacrylamide (3.08 g, 20 mmoles, 4 mole %) and ethanol (300 mL) were added. The mixture was purged with nitrogen for 10 min before the addition of a radical initiator, AIBN (330 mg, 2 mmoles). The mixture was heated to 70° C for 16 hours. At the end of reaction, reaction mixture was allowed to cool to room temperature and poured into a beaker containing isopropanol (1 liter). The polymer was precipitated out as a white solid, which was collected and ground to small pieces in a blender using isopropanol as a solvent. The pieces were collected by filtration and the polymer was dried under vacuum at 60°C for 2 days. The material was ground to a fine powder (84 g), which was used for the *in vitro* and *in vivo* studies.
The compounds of Table 7 were prepared using the above procedure.

**Table 7**

| Example No. | Acrylamide (mole %) | TMAEAC (mole %) | Octadecyl acrylate (mole %) | Methylenebisacrylamide (mole %) |
|---|---|---|---|---|
| 147 | 20 | 75 | 5 | 4 |
| 148 | 20 | 70 | 10 | 4 |
| 149 | 35 | 63 | 2 | 4 |
| 150 | 35 | 60 | 5 | 4 |
| 151 | 35 | 55 | 10 | 4 |
| 152 | 50 | 48 | 2 | 4 |
| 153 | 50 | 45 | 5 | 4 |
| 154 | 50 | 40 | 10 | 4 |
| 155 | 0 | 98 | 2 | 4 |
| 156 | 0 | 95 | 5 | 4 |
| 157 | 0 | 90 | 10 | 4 |

### EXAMPLE 158: PREPARATION OF METHYLENEBISACRYLAMIDE (4 MOLE %) CROSS-LINKED POLYMER OF ACRYLAMIDE (20 MOLE %)/TRIMETHYLAMINOETHYL ACRYLCHLORIDE Q SALT (TMAEAC) (75 MOLE %)/DODECYLACRYLATE (5 MOLE %)

To a 1-liter, three-necked flask equipped with condenser and stir bar, were added trimethylaminoethyl acrylchloride quaternary salt (TMAEAC) 50% aqueous solution (145.80 g of 50 % solution, 375 mmoles, 75 mole%), acrylamide (7.11 g, 100 mmoles, 20 mole%), dodecylacrylate (6.01 g, 25 mmoles, 5 mole%), methylenebisacrylamide (3.08 g, 20 mmoles, 4 mole %) and ethanol (300 mL). The mixture was purged with nitrogen for 10 min before the addition of a radical initiator, AIBN (330 mg, 2 mmoles). The mixture was heated to 70° C for 16 hours. The reaction mixture was allowed to cool to room temperature and poured into a beaker containing isopropanol (1 liter). The polymer was precipitated out as a white solid, which was collected and ground to small pieces in a blender using isopropanol as a solvent. The pieces were collected by filtration and the polymer was dried under vacuum at 60°C for 2 days. The material was ground to a fine powder (80 g), which was used for the *in vitro* and *in vivo* studies. The following cross-linked polymers were prepared.
The polymers of Table 8 were prepared using the above procedure.

**Table 8**

| Example No | Acrylamide (mole %) | TMAEAC (mole %) | Dodecylacrylate (mole %) | Methylenebisacrylamide ( mole %) |
|---|---|---|---|---|
| 159 | 20 | 70 | 10 | 4 |
| 160 | 20 | 55 | 25 | 4 |
| 161 | 35 | 60 | 5 | 4 |
| 162 | 35 | 55 | 10 | 4 |
| 163 | 35 | 40 | 25 | 4 |
| 164 | 50 | 45 | 5 | 4 |
| 165 | 50 | 40 | 10 | 4 |
| 166 | 50 | 25 | 25 | 4 |
| 167 | 0 | 95 | 5 | 4 |
| 168 | 0 | 90 | 10 | 4 |
| 169 | 0 | 75 | 25 | 4 |

### EXAMPLE 170: PREPARATION OF COPOLYMER OF METHACRYLAMIDE (20 MOL%)/TRIMETHYLAMINOETHYL METHACHLORIDE QUATERNARY SALT (TMAEMC) (78 MOL%)/OCTADECYL METHACRYLATE (2 MOL%)

To a 1 liter, three necked, round-bottomed flask equipped with condenser, stir bar, heating mantle (with J-Kem temperature controller) and nitrogen bubbler was added: trimethylammonioethyl methacrylate chloride (TMAEMC) ∼75% aqueous 87.19 g (420.39 mmol), octadecyl methacrylate (3.65 g, 10.80 mmol), methacrylamide (9.16 g, 107.76 mmol), and ethanol (300 mL). The total amount of monomer solids should be 100 g. Nitrogen was allowed to bubble through the room temperature monomer mixture for at least 20 minutes before adding 0.275 g AIBN [2,2'-azobis(2-methyl-propionitrile)]. At this point the nitrogen was set to blanket the mixture and the heat was turned on to 70°C. The reaction was allowed to heat for 22 hours at 70°C. While the polymer was still warm it was poured from the flask into a Nalgene bucket and allowed to stand for at least three hours in each of four 1-liter washings of isopropanol. Once the polymer became slightly rigid/rubbery it was broken up into small chunks using a blender (with isopropanol as the liquid). The granular product was filtered and washed with more isopropanol and placed in a crystallizing dish in a 70°C convection oven for two days. After this time, the product was removed and ground to a fine powder using a grinder and placed back in the oven for two more days. GPC analysis of octadecyl methacrylate containing polymers shows MW ranges form 100K-150K with polydispersities ranging from 2.5-5. The following table gives general mole percent compositions of polymers prepared in this fashion.
The polymers of Table 9 were prepared using the above procedure.

**Table 9**

| Example No. | Methacrylamide (mole %) | TMAEMC (mole %) | Octadecyl Methacrylate (mole%) |
|---|---|---|---|
| 171 | 0 | 98 | 2 |
| 172 | 0 | 95 | 5 |
| 173 | 0 | 90 | 10 |
| 174 | 0 | 85 | 15 |
| 175 | 0 | 80 | 20 |
| 170 | 20 | 78 | 2 |
| 176 | 20 | 75 | 5 |
| 177 | 20 | 70 | 10 |
| 178 | 35 | 63 | 2 |
| 179 | 35 | 60 | 5 |
| 180 | 35 | 55 | 10 |
| 181 | 50 | 48 | 2 |
| 182 | 50 | 45 | 5 |
| 183 | 50 | 40 | 10 |

### EXAMPLE 184: PREPARATION OF COPOLYMER OF METHACRYLAMIDE (20 MOL%)/TRIMETHYLAMINOETHYL METHACRY-CHLORIDE QUATERNARY SALT (TMAEMC) (75 MOL%)/DODECYL METHACRYLATE (5 MOL%)

To a 1-liter, three-necked, round-bottomed flask equipped with condenser, stir bar, heating mantle (with J-Kem temperature controller) and nitrogen bubbler was added: trimethylammonioethyl methacrylate chloride (TMAEMC) ∼75% aqueous (83.97 g, 404.86 mmol), dodecyl methacrylate (6.85 g, 26.96 mmol), methacrylamide (9.18 g, 108.0 mmol), and ethanol (300 mL). The total amount of monomer solids should be 100 g. Nitrogen was allowed to bubble through the room temperature monomer mixture for at least 20 minutes before adding 0.275g AIBN [2,2'-azobis(2-methyl-propionitrile)]. At this point, the nitrogen was set to blanket the mixture and the heat was turned on to 70°C. The reaction was allowed to heat for 22 hours at 70°C. While the polymer was still warm it was poured from the flask into a Nalgene bucket and allowed to stand for at least three hours in each of four 1-liter washings of isopropanol. Once the polymer became slightly rigid/rubbery it was broken up into small chunks using a blender (with isopropanol as the liquid). The granular product was filtered and washed with more isopropanol and placed in a crystallizing dish in a 70°C convection oven for two days. After this time, the product was removed and ground to a fine powder using a grinder and placed back in the oven for two more days. GPC analysis of dodecyl methacrylate containing polymers shows MW ranges form 170-190K with polydispersities ranging from 2.3-2.8. Table 10 gives general mole percent compositions of polymers prepared in this fashion.
The polymers of Table 10 were prepared using the above procedure.

**Table 10**

| Example No. | Methacrylamide (mole %) | TMAEMC (mole %) | Dodecyl metrhacrylate (mole %) |
|---|---|---|---|
| 185 | 0 | 95 | 5 |
| 186 | 0 | 90 | 10 |
| 187 | 0 | 75 | 25 |
| 184 | 20 | 75 | 5 |
| 188 | 20 | 70 | 10 |
| 189 | 20 | 55 | 25 |
| 190 | 35 | 60 | 5 |
| 191 | 35 | 55 | 10 |
| 192 | 35 | 40 | 25 |
| 193 | 50 | 45 | 5 |
| 194 | 50 | 40 | 10 |
| 195 | 50 | 25 | 25 |

### EXAMPLE 196: PREPARATION OF COPOLYMER OF TMAEMC (80 MOL%)/METHACRYLAMIDE (20 MOL%)

To a 1-liter, three-necked, round-bottomed flask equipped with condenser, stir bar, heating mantle (with J-Kem temperature controller) and nitrogen bubbler was added: trimethylammonioethyl methacrylate chloride (TMAEMC) ∼75% aqueous, (90.71 g, 437.36 mmol), methacrylamide (9.29 g, 109.29 mmol), and ethanol (300 mL). The total amount of monomer solids should be 100g. Nitrogen was allowed to bubble through the room temperature monomer mixture for at least 20 minutes before adding 0.275 g AIBN [2,2'-azobis(2-methyl-propionitrile)]. At this point, the nitrogen was set to blanket the mixture and the heat was turned on to 70°C. The reaction was allowed to heat for 22 hours at 70°C. While the polymer was still warm it was poured from the flask into a Nalgene bucket and allowed to stand for at least three hours in each of four 1-liter washings of isopropanol. Once the polymer became slightly rigid/rubbery it was broken up into small chunks using a blender (with isopropanol as the liquid). The granular product was filtered and washed with more isopropanol and placed in a crystallizing dish in a 70°C convection oven for two days. After this time the product was removed and ground to a fine powder using a grinder and placed back in the oven for two more days. Table 11 gives general mole percent compositions of polymers prepared in this fashion.
The polymers of Table 11 were prepared using the above procedure.

**Table 11**

| Example No. | Methacrylamide (mole %) | TMAEMC (mole %) |
|---|---|---|
| 197 | 0 | 100 |
| 196 | 20 | 80 |
| 198 | 35 | 65 |
| 199 | 50 | 50 |

### EXAMPLE 200: PREPARATION OF METHACRYLATE METHYLENEBISMETHACRYLAMIDE (2MOLE %) CROSS-LINKED POLYMER OF METHACRYLAMIDE (20 MOL%)/TMAEMC (78 MOL%)/ OCTADECYL METHACRYLATE (2 MOL%)

To a 1-liter, three-necked, two-part reaction flask equipped with condenser, mechanical stirrer, water bath, and nitrogen bubbler was added trimethylammonioethyl methacrylate chloride (TMAEMC), ∼75% aqueous (87.19 g, 420.39 mmol) octadecyl methacrylate (3.65 g,10.80 rnmol), methacrylamide (9.16 g, 107.76 mmol), and ethanol (400 mL). The total amount of monomer solids should be 100 g. To this was added an additional 2 mole percent (of total monomers) N,N'-methylenebismethacrylamide (1.96 g, 10.76 mmol). Nitrogen was allowed to bubble through the room temperature monomer mixture for at least 20 minutes before adding 0.275 g AIBN [2,2'-azobis(2-methyl-propionitrile)]. At this point, the nitrogen was set to blanket the mixture and the heat was turned on to 70°C. Once the polymer began to gel the stirring was turned off; total heating time at 70°C was approximately 5 hours. The polymer was then allowed cool down to room temperature and stand overnight. The gelled product was scooped out of the flask and swollen to a clear gel in a 500 mL isopropanol/1000 mL water mixture. The gel was washed 6X with 1000 mL isopropanol filtering over a 50-mesh sieve. Once the polymer became slightly rigid/rubbery it was broken up into small chunks using a blender (with isopropanol as the liquid). The product was filtered over a sieve, wrung out, and placed in a drying dish in a 70°C convection oven for two days. After this time, the product was removed and ground to a fine powder using a grinder and placed back in the oven for two more days in a glass crystallizing dish. Table 12 gives general mole percent compositions of polymers prepared in this fashion.
The polymers of Table 12 were prepared using the above procedure.

**Table 12**

| Example No. | Methacrylamide (mole%) | TMAEMC (mole %) | OctadecylMethacrylate (mole %) | Methylenebismethacrylamide (mole %) |
|---|---|---|---|---|
| 201 | 0 | 98 | 2 | 2 |
| 202 | 0 | 95 | 5 | 2 |
| 203 | 0 | 90 | 10 | 2 |
| 204 | 0 | 85 | 15 | 2 |
| 205 | 0 | 80 | 20 | 2 |
| 200 | 20 | 78 | 2 | 2 |
| 206 | 20 | 75 | 5 | 2 |
| 207 | 20 | 70 | 10 | 2 |
| 208 | 35 | 63 | 2 | 2 |
| 209 | 35 | 60 | 5 | 2 |
| 210 | 35 | 55 | 10 | i 2 |
| 211 | 50 | 48 | 2 | 2 |
| 212 | 50 | 45 | 5 | 2 |
| 213 | 50 | 40 | 10 | 2 |

### EXAMPLE 214: PREPARATION OF METHACRYLATEMETHYLENE-BISMETHACRYLAMIDE ( 2MOLE %) CROSS-LINKED POLYMER OF METHACRYLAMIDE (20 MOL%)/TMAEMC (75 MOL%)/ DODECYL METHACRYLATE (5 MOL%)

To a 1-liter, three-necked, two-part reaction flask equipped with condenser, mechanical stirrer, water bath, and nitrogen bubbler was added:
trimethylammonioethyl methacrylate chloride (TMAEMC), ∼75% aqueous solution (83.97 g , 404.86 mmol), dodecyl methacrylate (6.85 g, 26.96 mmol), methacrylamide (9.18g, 108.0 mmol) and ethanol (400 mL). The total amount of monomer solids should be 100 g. To this was added an additional 2 mole percent (of total monomers) N,N'-methylenebismethacrylamide (1.96 g, 10.79 mmol). Nitrogen was allowed to bubble through the room temperature monomer mixture for at least 20 minutes before adding 0.275 g AIBN [2,2'-azobis(2-methyl-propionitrile)]. At this point, the nitrogen was set to blanket the mixture and the heat was turned on to 70°C. Once the polymer began to gel the stirring was turned off; total heating time at 70°C was approximately 5 hours. The polymer was then allowed cool down to room temperature and stand overnight. The gelled product was scooped out of the flask and swollen to a clear gel in a 500 mL isopropanol/ 1000 mL water mixture. The gel was washed 6X with 1000 mL of isopropanol filtering over a 50-mesh sieve. Once the polymer became slightly rigid/rubbery it was broken up into small chunks using a blender (with isopropanol as the liquid). The product was filtered over a sieve, wrung out, and placed in a drying dish in a 70°C convection oven for two days. After this time, the product was removed and ground to a fine powder using a grinder and placed back in the oven for two more days in a glass crystallizing dish. Table 13 gives general mole percent compositions of polymers prepared in this fashion.

**Table 13**

| Example No. | Methacrylamide (mole%) | TMAEC (mole %) | Dodecyl methacrylate (mole%) | Methylenebismethacrylamide (mole %) |
|---|---|---|---|---|
| 215 | 0 | 95 | 5 | 2 |
| 216 | 0 | 90 | 10 | 2 |
| 217 | 0 | 75 | 25 | 2 |
| 214 | 20 | 75 | 5 | 2 |
| 218 | 20 | 70 | 10 | 2 |
| 219 | 20 | 55 | 25 | 2 |
| 220 | 35 | 60 | 5 | 2 |
| 221 | 35 | 55 | 10 | 2 |
| 222 | 35 | 40 | 25 | 2 |
| 223 | 50 | 45 | 5 | 2 |
| 224 | 50 | 40 | 10 | 2 |
| 225 | 50 | 25 | 25 | 2 |

### EXAMPLE 226: PREPARATION OF METHACRYLATEMETHYLENEBIS-METHACRYLAMIDE (2MOLE %) CROSS-LINKED POLYMER OF METHACRYLAMIDE (20 MOL%)/TMAEMC (80 MOL%)

To a 1-liter, three-necked, two-part reaction flask equipped with condenser, mechanical stirrer, water bath, and nitrogen bubbler was added:
trimethylammonioethyl methacrylate chloride (TMAEMC) ∼75% aqueous solution (90.71 g, 437.36 mmol), methacrylamide 9.29 g (109.29 mmol), and ethanol (400 mL). The total amount of monomer solids should be 100 g. To this was added an additional 2 mole percent (of total monomers) N,N'-methylenebismethacrylamide (1.989 g, 10.93 mmol). Nitrogen was allowed to bubble through the room temperature monomer mixture for at least 20 minutes before adding 0.275 g AIBN [2,2'-azobis(2-methyl-propionitrile)]. At this point, the nitrogen was set to blanket the mixture and the heat was turned on to 70°C. Once the polymer began to gel the stirring is turned off; total heating time at 70°C was approximately 5 hours. The polymer was then allowed cool down to room temperature and stand overnight. The gelled product was scooped out of the flask and swollen to a clear gel in a 500 mL isopropanol/ 1000 mL of water mixture. The gel was washed 6X with 1000 mL isopropanol filtering over a 50-mesh sieve. Once the polymer became slightly rigid/rubbery it was broken up into small chunks using a blender (with isopropanol as the liquid). The spongy product is filtered over a sieve, pressed dry, and placed in a drying dish in a 70°C convection oven for two days. After this time the product was removed and ground to a fine powder using a grinder and placed back in the oven for two more days in a glass crystallizing dish. Table 14 gives general mole percent compositions of polymers prepared in this fashion.

**Table 14**

| Example No. | Methacrylamide (mole %) | TMAEMC (mole%) | Methylenebismeth-Acrylamide (mole %) |
|---|---|---|---|
| 227 | 0 | 100 | 2 |
| 226 | 20 | 80 | 2 |
| 228 | 35 | 65 | 2 |
| 229 | 50 | 50 | 2 |

### METHOD FOR DETERMINING BINDING OF EMULSION PARTICLES BY LIPID-BINDING POLYMERS USING AN OLIVE OIL EMULSION WITH PHYSIOLOGICAL EMULSIFIERS

### PREPARATION OF OLIVE OIL EMULSION FOR LIPID-BINDING ASSAY

### EMULSIFIER SOLUTION

Egg yolk lecithin 2.54 mmol (2.00 g) and cholesterol 1.25 mmol (0.483 g) were dissolved in 100 mL of chloroform in a 1-liter, round-bottomed flask and the solvent was removed rapidly using a rotary evaporator. A coating of lecithin and cholesterol resulted, adhering to the walls of the flask. This film was held under vacuum for 12 hours. The sodium salts of the following bile acids were then added to the flask: glycocholic 1.217 g (2.496 mmol), taurocholic 0.895 g (1.664 mmol), glycodeoxycholic 1.766 g (3.744 mmol), taurodeoxycholic 1.302 g (2.496 mmol). An aqueous buffer consisting of 0.1M 2-[N-morpholino]ethanesulfonic acid (MES) and 0.1 M sodium chloride was prepared and the pH was adjusted with 50% NaOH to pH = 6.5. 1 liter of this aqueous buffer was added to the flask containing the coating of lecithin and cholesterol, and this mixture was stirred for 3-4 hours. During this time, the coating of lecithin and cholesterol was dispersed in solution. A cloudy solution resulted.

### EMULSION

In a 400 mL, thick-walled beaker, were mixed highly refined acid free olive oil 31.49 g, and oleic acid 3.51 g. The emulsifier solution described above was then added to bring the total weight of the mixture to 350 g. A 1-inch drying coated stir bar was added, and the mixture was stirred magnetically for 2-5 minutes. The mixture was then irradiated with 2 bursts of ultrasound (45 sec. each, with 2 minutes of magnetic stirring between bursts) using a Branson Sonifier 450 operated at maximum power with a 3/4" solid horn. The pH of the resulting emulsion was adjusted to 6.5 (at 20°C). The emulsion prepared in this way was used immediately in the fat binding test, but could be kept in a refrigerator (4°C) for a week.

When the physiological emulsion described above was mixed with test polymers, it was observed that a solid polymer/lipid complex would form in some cases. A test was devised to measure the quantity of lipid absorbed by the test polymers from the physiological emulsion.

### LIPID BINDING TEST

The test polymer (25 mg) was weighed into a tared 20 mL centrifuge filter cup with a 10 micron polypropylene mesh filter (Whatman VECTASPIN20™ centrifuge filter). The bottom of the filter cup was then sealed with tape to prevent solution from leaking out during the test. Using an analytical pipette, an aqueous buffer solution (3 mL) containing NaCl (0.1M), and MES (0.1M) at pH = 6.5 was added to the filter cup. The filter cup was inserted into its companion centrifuge tube and sealed with a cap. This assembly was agitated in an orbital shaker for at least I hour in order to dissolve or disperse the test polymer. The olive oil emulsion described above (15 mL) was then added to the filter cup using an analytical pipette. The cap was replaced, and the centrifuge tube shaken (250 rpm) on an orbital mixer for a period of one hour. The centrifuge filter device was then disassembled so that the tape could be removed from the bottom of the centrifuge filter cup. It was immediately reassembled and spun in a centrifuge at an RCF of 500 G, and at 25°C for 30 minutes. The centrifuge filter device was removed from the centrifuge and disassembled. The filter cup was weighed to obtain the weight gain of the wet polymer/lipid complex. This material was the removed from the filter cup with a spatula, and placed into a tared glass vial. The vial was weighed again to obtain the weight of the polymer/lipid sample. The vial was then placed into a centrifugal evaporator, and dried at 60°C under vacuum until a pressure of 0.15 Torr or less was achieved (8-18 hrs). The vial was removed and weighed to obtain the dry weight of the polymer/lipid complex sample. The amount of lipid absorbed by the original 25 mg polymer sample in the filter cup was then calculated. This gravimetric result was used as a measure for lipid binding by the polymer, and is listed in the accompanying table as lipid weight absorbed (g) per gram of polymer.

**TABLE 15**

| **Example No.** | **Lipid weight (g) absorbed by 1 gram of polymer** | **Example No.** | **Lipid weight (g) absorbed by 1 gram of polymer** |
|---|---|---|---|
| Chitosan | 2.2 | 92 | 10.9 |
| 10 | 5.5 | 94 | 2.8 |
| 13 | 6.8 | 96 | 4.7 |
| 14 | 20 | 97 | 4.5 |
| 15 | 44.9 | 98 | 1.7 |
| 16 | 3.4 | 99 | 5.7 |
| 52 | 3.3 | 100 | 3.3 |
| 54 | 4.6 | 101 | 11 |
| 55 | 3.8 | 102 | 13.4 |
| 66 | 5.9 | 103 | 4.3 |
| 67 | 5.9 | 104 | 16 |
| 69 | 4.5 | 105 | 2.1 |
| 70 | 4.1 | 107 | 7.7 |
| 72 | 5.8 | 108 | 6.6 |
| 75 | 4.5 | 109 | 5.1 |
| 76 | 3.9 | 110 | 6.1 |
| 78 | 3 | 111 | 4.8 |
| 79 | 3.6 | 112 | 3.8 |
| 80 | 12.6 | 113 | 60 |
| 81 | 3.1 | 114 | 58 |
| 83 | 10 | 115 | 58 |
| 84 | 2.7 | 116 | 59 |
| 85 | 11.3 | 117 | 59 |
| 86 | 2.1 | 118 | 60 |
| 87 | 6.5 | 119 | 54 |
| 88 | 3.1 | 120 | 59 |
| 89 | 6.9 | 121 | 60 |
| 90 | 4.5 | 122 | 56 |
| 91 | 6.2 | 123 | 54 |
| 124 | 50 | 180 | 57 |
| 125 | 59 | 181 | 68 |
| 126 | 52 | 182 | 60 |
| 127 | 56 | 183 | 51 |
| 128 | 66 | 184 | 65 |
| 129 | 62 | 185 | 7 |
| 130 | 60 | 186 | 67 |
| 131 | 59 | 187 | 27 |
| 132 | 58 | 188 | 2 |
| 133 | 59 | 189 | 43 |
| 134 | 49 | 190 | 10 |
| 135 | 49 | 191 | 2 |
| 136 | 43 | 192 | 68 |
| 137 | 57 | 193 | 67 |
| 138 | 64 | 194 | 61 |
| 140 | 63 | 195 | 64 |
| 141 | 66 | 196 | 16 |
| 166 | 48 | 197 | 8 |
| 167 | 59 | 198 | 13 |
| 168 | 61 | 199 | 5 |
| 169 | 39 | 202 | 9 |
| 172 | 61 | 203 | 6 |
| 173 | 33 | 204 | 15 |
| 174 | 39 | 205 | 7 |
| 175 | 65 | 206 | 3 |
| 176 | 15 | 207 | 22 |
| 177 | 37 | 208 | 4 |
| 178 | 21 | 209 | 3 |
| 179 | 24 | | |

### IN VIVO TESTING OF FAT-BINDING POLYMERS

The non-crosslinked and crosslinked fat-binding polymers of Examples 5, 6, 10, 72, 173 and Chitosan were evaluated for their ability to increase the excretion of fat in the feces, relative to the control group, in normal rats over a six-day period. Male Sprague-Dawley rats (five to six weeks of age) were individually housed and fed *ad libitum* a powdered "high-fat diet," consisting of standard rodent chow supplemented with 15% lard by weight. After feeding the animals this diet for five days, the animals were weighed and sorted into the treatment or control groups (4-6 animals per group, each group having equal mean body weights). Animals were treated for six days with the test compounds, which were added to the "high-fat diet" at concentrations (w/w) of 0.0% (control), 2.0 or 5.0 percent of the diet. In one study chitosan was evaluated for its effect on fecal fat excretion.

Rat fecal samples were collected on the final three days of the six days of drug treatment. The samples were freeze dried and ground to a fine powder. One half gram of sample was weighed and transferred to extraction cells. Samples were extracted in an accelerated solvent extractor (ASE 200 Accelerated Solvent Extractor, Dyonex Corporation, Sunnyvale, CA) with 95% ethanol, 5% water and 100 mM KOH. The sample was extracted in 17 minutes at 150°C and 1500 psi. An aliquot of extract was transferred to a test tube containing a molar excess of HCl. The sample was then evaporated and reconstituted in a detergent solution consisting of 2% Triton X-1200, 1% polyoxyethylene lauryl ether and 0.9% NaCl. Fatty acids were then quantitated enzymatically with a colorimetric kit (NEFAC, Wako Chemical GmbH, Neuss, Germany).
Table 16 contains values for fecal fat excretion as a percentage of ingested fat.

**Table 16:**

| ***IN VIVO* EFFICACY OF FAT-BINDING POLYMERS** | | |
|---|---|---|
| Example Identification | Dose (w/w percent of diet) | Fecal Fat Excretion % Of Ingested Fat |
| Example 6-PAA 3% XL, 10% C12 | 5 | 40 |
| Example 6-PAA 3% XL, 10% C12 | 2 | 12 |
| Example 5-PAA 9.4% XL | 2 | 22 |
| Example 10-PDA 4.5% XL | 2 | 30 |
| Example 72-PDA 4.5% XL, 11% C12 | 2 | 29 |
| Example 173-TMAEMC (90 mol%) + octadecyl methacrylate(10 mol%) | 2 | 20 |
| Chitosan | 2 | 8 |

Fecal Fat/Consumed Fat was calculated as follows: Fatty acid concentration from the enzymatic assay was expressed as mmol/mL. The mmol/mL of fatty acid was then multiplied by the number of mL of extract generated from 500 mg of sample to give the total mmol of fatty acid. The value for the total mmol of fatty acid was converted to total mg of fatty acid using the average molecular weight of medium to long chain fatty acid (270 D). The value was corrected for any dilutions made during sample workup. When results are expressed as mg/gm of feces, the total mg of fatty acids is multiplied by 2. When results were expressed as total mg of fatty acid excreted in 24 hours, the mg/gm of feces value was multiplied by fecal weight in grams excreted in 24 hours. When the results were expressed as excreted fat as a % of that consumed in 24 hours, the total weight of fat excreted in 24 hours was divided by the weight of fatty acids consumed over 24 hours and multiplied by 100.

## Claims

1. A pharmaceutical composition or kit of parts for treating obesity in a mammal by oral administration to the mammal comprising an effective amount of one or more fat-binding polymers and one or more lipase inhibitors.

2. The composition or kit of parts of Claim 1 wherein the fat-binding polymer is a synthetic polymer.

3. The composition or kit of parts of Claim 1 or Claim 2 wherein the fat-binding polymer is selected from the group consisting of polyalkylacrylates, polyacrylamides, polyalkylmethacrylates, polymethacrylamides, poly-N-alkylacrylamides, poly-N-alkylmethacrylamides, substituted derivatives thereof and copolymers thereof.

4. The composition or kit of parts of Claim 3 wherein the fat-binding polymer is poly(dimethylamino propylacrylamide), poly(trimethylammonium ethylacrylate), poly(brimethylammoniumethyl methacrylate), poly(trimethylammoniumpropyl acrylamide), poly(dodecyl acrylate), poly(octadecyl acrylate), poly(octadecyl methacrylate) or copolymers thereof.

5. The composition or kit of parts of Claim 3 or Claim 4 wherein the polymer is crosslinked by a multifunctional co-monomer.

6. The composition or kit of parts of Claim 5 wherein the multifunctional co-monomer is selected from the group consisting of diacrylates, triacrylates, tetraacrylates, dimethacrylates, diacrylamides, dimethacrylamides, diallylacrylamides and polyvinylarenes.

7. The composition or kit of parts of Claim 6 wherein the multifunctional comonomer is selected from the group consisting of ethylene glycol diacrylate, propylene glycol diacrylate, butylene glycol diacrylate, ethylene glycoi dimethacrylate, butylene glycol dimethacrylate, methylene bis(methacrylamide), ethylene bis(acrylamide), ethylene bis(methacrylamide), ethylidene bis(acrylamide), ethylidene bis(methacrylamide), bisphenol A dimethacrylate, bisphenol A diacrylate, pentaerythritol tetraacrylate, trimethylolpropane triacrylate and divinylbenzene.

8. The composition or kit of parts of any one of Claims 5 to 7, wherein the amount of crosslinking agent is between 0.5 and 25 weight percent based on the combined weight of crosslinking agent and monomers.

9. The composition or kit of parts of any one of Claims 1 to 8, wherein the lipase inhibitor is selected from lipstatin, tetrahydrolipstatin or a combination thereof.

10. The composition or kit of parts of Claim 1 wherein the fat-binding polymer is a synthetic amine polymer.

11. The composition or kit of parts of Claim 10 wherein the amine polymer is selected from the group consisting of poly(allylamine), poly(ethyleneimine), poly(vinylamine), poly(diallylamine), and poly(diallylmethylamine).

12. The composition or kit of parts of Claim 11 wherein the amine polymer is crosslinked by means of a multifunctional crosslinking agent, said agent being present in an amount from 0.5%-25% weight, based upon the combined weight of monomer and crosslinking agent.

13. The composition or kit of parts of any one of Claims 10 to 12 wherein the amine polymer further comprises one or more hydrophobic regions bound to a portion of the amine nitrogens.

14. The composition or kit of parts of Claim 13 wherein the hydrophobic regions are bound to between 1 and 60 percent of the amine nitrogens.

15. The composition or kit of parts of Claim 14 wherein the hydrophobic regions are bound to between 1 and 30 percent of the amine nitrogens.

16. The composition or kit of parts of any one of Claims 13 to 15, wherein the hydrophobic region is a substituted or unsubstituted, normal, branched or cyclic alkyl group having at least four carbons.

17. The composition or kit of parts of Claim 16 wherein the hydrophobic region is an alkyl group of between four and thirty carbons.

18. The composition or kit of parts of Claim 17 wherein the hydrophobic region is an alkyl group of 6 carbons.

19. The composition or kit of parts of Claim 17 wherein the hydrophobic region is an alkyl group of 8 carbons.

20. The composition or kit of parts of Claim 17 wherein the hydrophobic region is an alkyl group of 10 carbons.

21. The composition or kit of parts of Claim 17 wherein the hydrophobic region is an alkyl group of 12 carbons.

22. The composition or kit of parts of Claim 17 wherein the hydrophobic region is an alkyl group of 18 carbons.

23. The composition or kit of parts of any one of Claims 10 to 22, wherein the amine polymer further comprises one or more positively charged regions bound to a portion of the amine nitrogens.

24. The composition or kit of parts of Claim 23 wherein the positively charged region comprises a quaternary amine-containing moiety having the following formula: 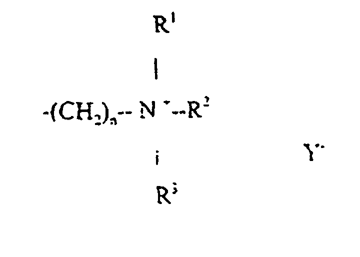
R¹, R² and R³ represent hydrogen or an alkyl group, wherein each R, independently, is a normal or branched, substituted or unsubstituted, normal, branched or cyclic alkyl group having a carbon atom chain length of between about one to about twenty-four carbon atoms;
n is an integer having a value of three or more; and
Y is a negatively-charged counterion.

25. The composition or kit of parts of Claim 1 wherein the fat-binding polymer comprises a monomer of the formula 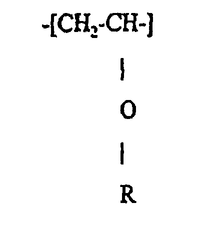 wherein R is a hydrophobic region.

26. The composition or kit of parts of Claim 25 wherein the hydrophobic region comprises a substituted or unsubstituted, normal, branched or cyclic alkyl group having at least about four carbons.

27. The composition or kit of parts of Claim 25 or Claim 26 wherein the hydrophobic region comprises an alkyl group of between four and thirty carbons.

28. The composition or kit of parts of Claim 26 wherein the hydrophobic region comprises an alkyl group of 6 carbons.

29. The composition or kit of parts of Claim 1 wherein the fat-binding polymer is substituted with a lipase inhibitor.

30. The use of an effective amount of one or more fat-binding polymers in the manufacture of a medicament for simultaneous or sequential administration with one or more lipase inhibitors for treating obesity in a mammal by oral administration.

31. A pharmaceutical composition or kit of parts for reducing the absorption of dietary fat in a mammal by oral administration to the mammal comprising a therapeutic amount of one or more fat-binding polymers in combination with one or more lipase inhibitors.

32. The use of a therapeutic amount of one or more fat-binding polymers, in the manufacture of a medicament for treating steatorrhea by oral administration.

33. The use of an effective amount of one or more fat-binding polymers in the manufacture of a medicament for simultaneous or sequential administration with one or more lipase inhibitors, for treating hypertriglyceridemia in a mammal by oral administration.

34. The composition or kit of parts of Claim 31 or the use of any one of Claims 30, 32 or 33, wherein the fat-binding polymer is a synthetic polymer.

## Patentansprüche

1. Pharmazeutische Zusammensetzung oder Kit-of-parts bzw. mehrteiliges Kit zur Behandlung von Fettleibigkeit bei einem Säugetier durch orale Verabreichung an das Säugetier, wobei die Zusammensetzung bzw. das Kit eine wirksame Menge von einem oder mehreren fettbindenden Polymeren und einen oder mehrere Lipaseinhibitioren umfasst.

2. Zusammensetzung oder Kit-of-parts nach Anspruch 1, wobei das fettbindende Polymer ein synthetisches Polymer ist.

3. Zusammensetzung oder Kit-of-parts nach Anspruch 1 oder Anspruch 2, wobei das fettbindende Polymer ausgewählt ist aus der Gruppe von Polyalkylacrylaten, Polyacrylamiden, Polyalkylmethacrylaten, Polymethacrylamiden, Poly-N-alkylacrylamiden, Poly-N-alkylmethacrylamiden, substituierten Derivaten derselben und Copolymeren derselben.

4. Zusammensetzung oder Kit-of-parts nach Anspruch 3, wobei das fettbindende Polymer Poly(dimethylaminopropylacrylamid), Poly(trimethylammoniumethylacrylat), Poly(trimethylammoniumethylmethacrylat), Poly(trimethylammoniumpropylacrylamid), poly(dodecylacrylat), Poly(octadecylacrylat), Poly(octadecylmethacrylat) oder ein Copolymer derselben ist.

5. Zusammensetzung oder Kit-of-parts nach Anspruch 3 oder Anspruch 4, wobei das Polymer durch ein mehrfunktionelles Comonomer vernetzt ist.

6. Zusammensetzung oder Kit-of-parts nach Anspruch 5, wobei das mehrfunktionelle Comonomer ausgewählt ist aus der Gruppe von Diacrylaten, Triacrylaten, Tetraacrylaten, Dimethacrylaten, Diacrylamiden, Dimethacrylamiden, Diallylacrylamiden und Polyvinylarenen.

7. Zusammensetzung oder Kit-of-parts nach Anspruch 6, wobei das mehrfunktionelle Comonomer ausgewählt ist aus der Gruppe von Ethylenglykoldiacrylat, Propylenglykoldiacrylat, Butylenglykoldiacrylat, Ethylenglykoldimethacrylat, Butylenglykoldimethacrylat, Methylenbis(methacrylamid), Ethylenbis(acrylamid), Ethylenbis (methacrylamid), Ethylidenbis(acrylamid), Ethylidenbis(methacrylamid), Bisphenol-A-dimethacrylat, Bisphenol-A-diacrylat, Pentaerythrittetraacrylat, Trimethylolpropantriacrylat und Divinylbenzol.

8. Zusammensetzung oder Kit-of-parts nach einem der Ansprüche 5 bis 7, wobei die Menge des Vernetzungsmittels zwischen 0,5 und 25 Gew.-%, bezogen auf das Gesamtgewicht von vernetzungemittel und Monomeren, beträgt.

9. Zusammensetzung oder Kit-of-parts nach einem der Ansprüche 1 bis 8, wobei der Lipaseinhibitor ausgewählt ist aus Lipstatin, Tetrahydrolipstatin oder einer Kombination derselben.

10. Zusammensetzung oder Kit-of-parts nach Anspruch 1, wobei das fettbindende Polymer ein synthetisches Aminpolymer ist.

11. Zusammensetzung oder Kit-of-parts nach Anspruch 10, wobei das Aminpolymer ausgewählt ist aus der Gruppe von Poly(allylamin), Poly(ethylenimin), Poly(vinylamin), Poly(diallylamin) und Poly(diallylmethylamin).

12. Zusammensetzung oder Kit-of-parts nach Anspruch 11, wobei das Aminpolymer mittels eines mehrfunktionellen Vernetzungsmittels vernetzt ist, wobei das Mittel in einer Menge von 0,5-25 Gew.-%, bezogen auf das Gesamtgewicht von Monomer und Vernetzungsmittel, vorhanden ist.

13. Zusammensetzung oder Kit-of-parts nach einem der Ansprüche 10 bis 12, wobei das Aminpolymer ferner einen oder mehrere hydrophobe Bereiche, die an einen Teil der Aminstickstoffatome gebunden sind, umfasst.

14. Zusammensetzung oder Kit-of-parts nach Anspruch 13, wobei die hydrophoben Bereiche an zwischen 1 und 60 % der Aminstickstoffatome gebunden sind.

15. Zusammensetzung oder Kit-of-parts nach Anspruch 14, wobei die hydrophoben Bereiche an zwischen 1 und 30 % der Aminstickstoffatome gebunden sind.

16. , Zusammensetzung oder Kit-of-parts nach einem der Ansprüche 13 bis 15, wobei der hydrophobe Bereich eine substituierte oder unsubstituierte, normale, verzweigte oder cyclische Alkylgruppe mit mindestens vier Kohlenstoffatomen ist.

17. Zusammensetzung oder Kit-of-parts nach Anspruch 16, wobei der hydrophobe Bereich eine Alkylgruppe mit zwischen vier und dreißig Kohlenstoffatomen ist.

18. Zusammensetzung oder Kit-of-parts nach Anspruch 17, wobei der hydrophobe Bereich eine Alkylgruppe mit 6 Kohlenstoffatomen ist.

19. Zusammensetzung oder Kit-of-parts nach Anspruch 17, wobei der hydrophobe Bereich eine Alkylgruppe mit 8 Kohlenstoffatomen ist.

20. Zusammensetzung oder Kit-of-parts nach Anspruch 17, wobei der hydrophobe Bereich eine Alkylgruppe mit 10 Kohlenstoffatomen ist.

21. Zusammensetzung oder Kit-of-parts nach Anspruch 17, wobei der hydrophobe Bereich eine Alkylgruppe mit 12 Kohlenstoffatomen ist.

22. Zusammensetzung oder Kit-of-parts nach Anspruch 17, wobei der hydrophobe Bereich eine Alkylgruppe mit 18 Kohlenstoffatomen ist.

23. Zusammensetzung oder Kit-of-parts nach einem der Ansprüche 10 bis 22, wobei das Aminpolymer ferner einen oder mehrere positiv geladene Bereiche, die an einen Teil der Aminstickstoffatome gebunden sind, umfasst.

24. Zusammensetzung oder Kit-of-parts nach Anspruch 23, wobei der positiv geladene Bereich eine ein quaternäres Amin enthaltende Einheit mit der folgenden Formel 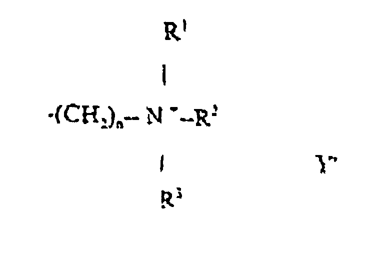 worin R¹, R² und R³ für Wasserstoff oder eine Alkylgruppe stehen, wobei jedes R in unabhängiger Weise eine normale oder verzweigte, substituierte oder unsubstituierte, normale, verzweigte oder cyclische Alkylgruppe mit einer Kohlenatoffatomkettenlänge von zwischen etwa einem bis etwa vierundzwanzig Kohlenstoffatomen ist;
n eine ganze Zahl mit einem Wert von drei oder mehr ist; und
Y ein negativ geladenes Gegenion ist, umfasst.

25. Zusammensetzung oder Kit-of-parts nach Anspruch 1, wobei das fettbindende Polymer ein Monomer der Formel 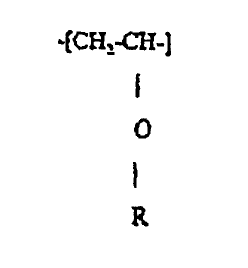 worin R ein hydrophober Bereich ist, umfasst.

26. Zusammensetzung oder Kit-of-parts nach Anspruch 25, wobei der hydrophobe Bereich eine substituierte oder unsubstituierte, normale, verzweigte oder cyclische Alkylgruppe mit mindestens etwa vier Kohlenstoffatomen umfasst.

27. Zusammensetzung oder Kit-of-parts nach Anspruch 25 oder Anspruch 26, wobei der hydrophobe Bereich eine Alkylgruppe mit zwischen vier und dreißig Kohlenstoffatomen umfasst.

28. Zusammensetzung oder Kit-of-parts nach Anspruch 26, wobei der hydrophobe Bereich eine Alkylgruppe mit sechs Kohlenstoffatomen umfasst.

29. Zusammensetzung oder Kit-of-parts nach Anspruch 1, wobei das fettbindende Polymer mit einem Lipaseinhibitor substituiert ist.

30. Verwendung einer wirksamen Menge von einem oder mehreren fettbindenden Polymeren bei der Herstellung eines Medikaments zur gleichzeitigen oder aufeinanderfolgenden Verabreichung mit einem oder mehreren Lipaseinhibitoren zur Behandlung von Fettleibigkeit bei einem Säugetier durch orale Verabreichung.

31. Pharmazeutische Zusammensetzung oder Kit-of-parts zur Verminderung der Absorption von Fett der täglichen Nahrung bei einem Säugetier durch orale Verabreichung an das Säugetier, wobei die Zusammensetzung eine therapeutische Menge von einem oder mehreren fettbindenden Polymeren in Kombination mit einem oder mehreren Lipaseinhibitoren umfasst.

32. Verwendung einer therapeutischen Menge von einem oder mehreren fettbindenden Polymeren bei der Herstellung eines Medikaments zur Behandlung von steatorrhoe durch orale Verabreichung.

33. Verwendung einer wirksamen Menge von einem oder mehreren fettbindenden Polymeren bei der Herstellung eines Medikaments zur gleichzeitigen oder aufeinanderfolgenden Verabreichung mit einem oder mehreren Lipaseinhibitoren zur Behandlung von Hypertriglyceridaemie bei einem Säugetier durch orale Verabreichung.

34. Zusammensetzung oder Kit-of-parts nach Anspruch 31 oder Verwendung nach einem der Ansprüche 30, 32 oder 33, wobei das fettbindende Polymer ein synthetisches Polymer ist.

## Revendications

1. Composition pharmaceutique ou collection (« kit of parts ») pour le traitement de l'obésité chez un mammifère par administration orale au mammifère comprenant une quantité efficace d'un ou de plusieurs polymères fixant les graisses et un ou plusieurs inhibiteurs de lipase.

2. Composition ou collection en plusieurs parties selon la revendication 1, dans laquelle le polymère fixant les graisses est un polymère synthétique.

3. Composition ou collection en plusieurs parties selon la revendication 1 ou la revendication 2, dans laquelle le polymère fixant les graisses est choisi dans le groupe constitué des poly(acrylates d'alkyle), polyacrylamides, poly(méthacrylates d'alkyle), polyméthacrylamides, poly-N-alkylacrylamides, poly-N-alkylméthacrylamides, dérivés substitués de ceux-ci et copolymères de ceux-ci.

4. Composition ou collection en plusieurs parties selon la revendication 3, dans laquelle le polymère fixant les graisses est un poly(diméthylamino propylacrylamide), poly(triméthylammonium acrylate d'éthyle), poly (triméthylammonium méthacrylate de méthyle), poly(triméthylammoniumpropylacrylamide), poly(acrylate de dodécyle), poly(acrylate d'octadécyle), poly(méthacrylate d'octadécyle) ou des copolymères de ceux-ci.

5. Composition ou collection en plusieurs parties selon la revendication 3 ou la revendication 4, dans laquelle le polymère est réticulé par un co-monomère multifonctionnel.

6. Composition ou collection en plusieurs parties selon la revendication 5, dans laquelle le co-monomère multifonctionnel est choisi dans le groupe constitué des diacrylates, triacrylates, tétraacrylates, diméthacrylates, diacrylamides, diméthacrylamides, diallylacrylamides et polyvinylarènes.

7. Composition ou collection en plusieurs parties selon la revendication 6, dans laquelle le co-monomère multifonctionnel est choisi dans le groupe constitué par l'éthylène glycol diacrylate, le propylène glycol diacrylate, le butylène glycol diacrylate, l'éthylène glycol diméthacrylate, le butylène glycol diméthacrylate, le méthylène bis(méthacrylamide), l'éthylène bis(acrylamide), l'éthylène bis(méthacrylamide), l'éthylidène bis (acrylamide), l'éthylidène bis(méthacrylamide), le diméthacrylate de bisphénol A, le diacrylate de bisphénol A, le tétraacrylate de penta-érythritol, le triacrylate de triméthylolpropane et le divinylbenzène.

8. Composition ou collection en plusieurs parties selon l'une quelconque des revendications 5 à 7, dans laquelle la quantité d'agent de réticulation est entre 0,5 et 25 pour cent en poids basé sur le poids combiné de l'agent de réticulation et des monomères.

9. Composition ou collection en plusieurs parties selon l'une quelconque des revendications 1 à 8, dans laquelle l'inhibiteur de lipase est choisi parmi la lipstatine, la tétrahydrolipstatine ou une combinaison de celles-ci.

10. Composition ou collection en plusieurs parties selon la revendication 1, dans laquelle le polymère fixant les graisses est un polymère aminé synthétique.

11. Composition ou collection en plusieurs parties selon la revendication 10, dans laquelle le polymère aminé est choisi dans le groupe constitué par la poly(allylamine), la poly(éthylèneimine), la poly(vinylamine), la poly(diallylamine), et la poly(diallylméthylamine).

12. Composition ou collection en plusieurs parties selon la revendication 11, dans laquelle le polymère aminé est réticulé au moyen d'un agent de réticulation multifonctionnel, ledit agent étant présent dans une quantité de 0,5% à 25% en poids, basé sur le poids combiné du monomère et de l'agent de réticulation.

13. Composition ou collection en plusieurs parties selon l'une quelconque des revendications 10 à 12, dans laquelle le polymère aminé comprend en outre une ou plusieurs régions hydrophobes liées à une partie des azotes des amines.

14. Composition ou collection en plusieurs parties selon la revendication 13, dans laquelle les régions hydrophobes sont liées à entre 1 et 60 pour cent des azotes des amines.

15. Composition ou collection en plusieurs parties selon la revendication 14, dans laquelle les régions hydrophobes sont liées à entre 1 et 30 pour cent des azotes des amines.

16. Composition ou collection en plusieurs parties selon l'une quelconque des revendications 13 à 15, dans laquelle la région hydrophobe est un groupe alkyle substitué ou non substitué, normal, ramifié ou cyclique ayant au moins quatre carbones.

17. Composition ou collection en plusieurs parties selon la revendication 16, dans laquelle la région hydrophobe est un groupe alkyle de entre quatre et trente carbones.

18. Composition ou collection en plusieurs parties selon la revendication 17, dans laquelle la région hydrophobe est un groupe alkyle de 6 carbones.

19. Composition ou collection en plusieurs parties selon la revendication 17, dans laquelle la région hydrophobe est un groupe alkyle de 8 carbones.

20. Composition ou collection en plusieurs parties selon la revendication 17, dans laquelle la région hydrophobe est un groupe alkyle de 10 carbones.

21. Composition ou collection en plusieurs parties selon la revendication 17, dans laquelle la région hydrophobe est un groupe alkyle de 12 carbones.

22. Composition ou collection en plusieurs parties selon la revendication 17, dans laquelle la région hydrophobe est un groupe alkyle de 18 carbones.

23. Composition ou collection en plusieurs parties selon l'une quelconque des revendications 10 à 22, dans laquelle le polymère aminé comprend en outre une ou plusieurs régions chargées positivement liées à une partie des azotes des amines.

24. Composition ou collection en plusieurs parties selon la revendication 23, dans laquelle la région chargée positivement comprend un fragment contenant une amine quaternaire ayant la formule suivante : 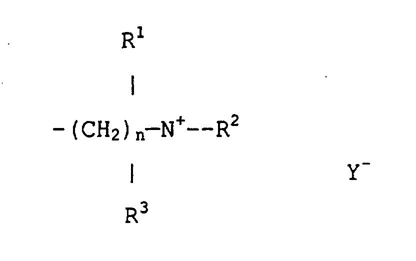 R¹, R², R³ représentent l'hydrogène ou un groupe alkyle, dans laquelle chaque R, indépendamment, est un groupe alkyle normal ou ramifié, substitué ou non substitué, normal, ramifié ou cyclique ayant une chaîne d'atomes de carbone longue d'environ un à environ vingt-quatre atomes de carbones ;
n est un nombre entier ayant une valeur de trois ou plus ; et
Y est un contre-ion chargé négativement.

25. Composition ou collection en plusieurs parties selon la revendication 1, dans laquelle le polymère fixant les graisses comprend un monomère de formule : 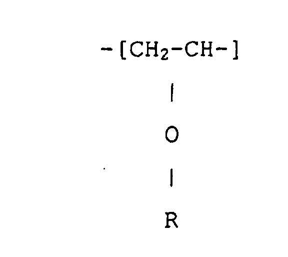 où R est une région hydrophobe.

26. Composition ou collection en plusieurs parties selon la revendication 25, dans laquelle la région hydrophobe comprend un groupe alkyle substitué ou non substitué, normal, ramifié ou cyclique ayant au moins environ quatre carbones.

27. Composition ou collection en plusieurs parties selon la revendication 25 ou la revendication 26, dans laquelle la région hydrophobe comprend un groupe alkyle de entre quatre et trente carbones.

28. Composition ou collection en plusieurs parties selon la revendication 26, dans laquelle la région hydrophobe comprend un groupe alkyle de 6 carbones.

29. Composition ou collection en plusieurs parties selon la revendication 1, dans laquelle le polymère fixant les graisses est substitué avec un inhibiteur de lipase.

30. Utilisation d'une quantité efficace d'un ou de plusieurs polymères fixant les graisses dans la fabrication d'un médicament pour une administration simultanée ou successive avec un ou plusieurs inhibiteurs de lipase pour le traitement de l'obésité chez un mammifère par administration orale.

31. Composition pharmaceutique ou collection en plusieurs parties pour la réduction de l'absorption de graisse alimentaire chez un mammifère par administration orale au mammifère comprenant une quantité thérapeutique d'un ou de plusieurs polymères fixant les graisses en combinaison avec un ou plusieurs inhibiteurs de lipase.

32. Utilisation d'une quantité thérapeutique d'un ou de plusieurs polymères fixant les graisses, dans la fabrication d'un médicament pour le traitement de la stéatorrhée par administration orale.

33. Utilisation d'une quantité efficace d'un ou de plusieurs polymères fixant les graisses dans la fabrication d'un médicament pour une administration simultanée ou successive avec un ou plusieurs inhibiteurs de lipase, pour le traitement de l'hypertriglycéridémie chez un mammifère par administration orale.

34. Composition ou collection en plusieurs parties selon la revendication 31 ou utilisation selon l'une quelconque des revendications 30, 32 ou 33, dans laquelle le polymère fixant les graisses est un polymère synthétique.
